# EUROPEAN PATENT SPECIFICATION

(11) **EP 2 021 357 B1**
(45) Date of publication and mention of the grant of the patent: **08.06.2011**
(21) Application number: 06755971.6
(22) Date of filing: 26.04.2006
(51) Int. Cl.: C07K 14/195, C12P 21/00

(54) **CYCLODIPEPTIDE SYNTHETASES AND THEIR USE FOR SYNTHESIS OF CYCLO(LEU-LEU) CYCLODIPEPTIDE**
CYCLODIPEPTIDSYNTHETASEN UND IHRE VERWENDUNG ZUR SYNTHESE VON CYCLO(LEU-LEU)CYCLODIPEPTID
CYCLODIPEPTIDE SYNTHETASES ET LEUR UTILISATION POUR LA SYNTHESE DE CYCLODIPEPTIDE CYCLO(LEU-LEU)

(43) Date of publication of application: 11.02.2009
(73) Proprietor: Commissariat à l'Énergie Atomique et aux Énergies Alternatives, 75015 Paris (FR); Kyowa Hakko Bio Co., Ltd, Chiyoda-ku Tokyo 100-8185 (JP); UNIVERSITE PARIS-SUD (PARIS XI), 91405 Orsay Cédex (FR); Centre National de la Recherche Scientifique, 75016 Paris Cedex 16 (FR)
(72) Inventor: GONDRY, Muriel, F-91470 Limours (FR); THAI, Robert, F-91620 Nozay (FR); BELIN, Pascal, F-91430 Igny (FR); GENET, Roger, F-91470 Limours (FR); PERNODET, Jean-Luc, F-94230 Cachan (FR)
(74) Representative: Thomas, Dean
(86) International application number: PCT/IB2006/001849
(87) International publication number: WO 2007/122446

(56) References cited:
- WO-A2-2004/011609
- FR-A1- 2 841 260
- DATABASE EMBL [Online] 20 November 2003 (2003-11-20), DUCHAUD E. ET AL.: XP002414406 retrieved from EBI Database accession no. ACF70114
- LAUTRU S ET AL: "The Albonoursin Gene Cluster of S. noursei - Biosynthesis of Diketopiperazine Metabolites Independent of Nonribosomal Peptide Synthetases" CHEMISTRY AND BIOLOGY, CURRENT BIOLOGY, LONDON, GB, vol. 9, no. 12, December 2002 (2002-12), pages 1355-1364, XP004545193 ISSN: 1074-5521
- RHEE K-H: "Cyclic dipeptides exhibit synergistic, broad spectrum antimicrobial effects and have anti-mutagenic properties" INTERNATIONAL JOURNAL OF ANTIMICROBIAL AGENTS, AMSTERDAM, NL, vol. 24, no. 5, November 2004 (2004-11), pages 423-427, XP004617403 ISSN: 0924-8579
- GONDRY M ET AL: "Cyclic dipeptide oxidase from Streptomyces noursei: Isolation, purification and partial characterization of a novel, amino acyl alpha,beta-dehydrogenase" EUROPEAN JOURNAL OF BIOCHEMISTRY, BERLIN, DE, vol. 268, March 2001 (2001-03), pages 1712-1721, XP002242439 ISSN: 0014-2956

### SEQUENCE LISTING

<110> Commissariat à l'Energie Atomique Kyowa Hakko Kogyo co., Ltd. Université Paris sud 11
   GONDRY, Muriel
   THAÏ, Robert
   BELIN, Pascal
   GENET, Roger
   PERNODET, Jean-Luc
<120> CYCLODIPEPTIDE SYNTHETASES AND THEIR USE FOR SYNTHESIS OF CYCLO(LEU-LEU) CYCLODIPEPTIDE
<130> BLOcp263/156
<160> 29
<170> Patentln version 3.3
<210> 1
   <211> 745
   <212> DNA
   <213> Bacillus subtilis
<220>
   <221> CDS
   <222> (1)..(744)
<400> 1
   atg acc gga atg gta acg gaa aga agg tct gtg cat ttt att gct gag      48
   Met Thr Gly Met Val Thr Glu Arg Arg Ser Val His Phe Ile Ala Glu
   1               5                   10                  15
   gca tta aca gaa aac tgc aga gaa ata ttt gaa cgg cgc agg cat gtt      96
   Ala Leu Thr Glu Asn Cys Arg Glu Ile Phe Glu Arg Arg Arg His Val
               20                  25                  30
   ttg gtg ggg atc agc cca ttt aac agc agg ttt tca gag gat tat att     144
   Leu Val Gly Ile Ser Pro Phe Asn Ser Arg Phe Ser Glu Asp Tyr Ile
           35                  40                  45
   tac aga tta att gga tgg gcg aaa gct caa ttt aaa agc gtt tca gtt     192
   Tyr Arg Leu Ile Gly Trp Ala Lys Ala Gln Phe Lys Ser Val Ser Val
       50                  55                  60
   tta ctt gca ggg cat gag gcg gct aat ctt cta gaa gcg ctt gga act     240
   Leu Leu Ala Gly His Glu Ala Ala Asn Leu Leu Glu Ala Leu Gly Thr
   65                  70                  75                  80
   ccg aga gga aag gct gaa cga aaa gta agg aaa gag gta tca cga aac     288
   Pro Arg Gly Lys Ala Glu Arg Lys Val Arg Lys Glu Val Ser Arg Asn
                   85                  90                  95
   agg aga ttt gca gaa aga gcc ctt gtg gct cat ggc ggg gat ccg aag     336
   Arg Arg Phe Ala Glu Arg Ala Leu Val Ala His Gly Gly Asp Pro Lys
               100                 105                 110
   gcg att cat aca ttt tct gat ttt ata gat aac aaa gcc tac cag ctg     384
   Ala Ile His Thr Phe Ser Asp Phe Ile Asp Asn Lys Ala Tyr Gln Leu
           115                 120                 125
   ttg aga caa gaa gtt gaa cat gca ttt ttt gag cag cct cat ttt cga     432
   Leu Arg Gln Glu Val Glu His Ala Phe Phe Glu Gln Pro His Phe Arg
       130                 135                 140
   cat gct tgt ttg gac atg tct cgt gaa gcg ata atc ggg cgt gcg cgg     480
   His Ala Cys Leu Asp Met Ser Arg Glu Ala Ile Ile Gly Arg Ala Arg
   145 150 155 160
   ggc gtc agt ttg atg atg gaa gaa gtc agt gag gat atg ctg aat ttg 528
   Gly Val Ser Leu Met Met Glu Glu Val Ser Glu Asp Met Leu Asn Leu
   165 170 175
   gct gtg gaa tat gtc ata gct gag ctg ccg ttt ttt atc gga gct ccg 576
   Ala Val Glu Tyr Val Ile Ala Glu Leu Pro Phe Phe Ile Gly Ala Pro
   180 185 190
   gat att tta gag gtg gaa gag aca ctc ctt gct tat cat cgt ccg tgg 624
   Asp Ile Leu Glu Val Glu Glu Thr Leu Leu Ala Tyr His Arg Pro Trp
   195 200 205
   aag ctg ggt gag aag atc agt aac cat gaa ttt tct att tgt atg cgg 672
   Lys Leu Gly Glu Lys Ile Ser Asn His Glu Phe Ser Ile Cys Met Arg
   210 215 220
   ccg aat caa ggg tat ctc att gta cag gaa atg gcg cag atg ctt tct 720
   Pro Asn Gln Gly Tyr Leu Ile Val Gln Glu Met Ala Gln Met Leu Ser
   225 230 235 240
   gag aaa cgg atc aca tct gaa gga t 745
   Glu Lys Arg Ile Thr Ser Glu Gly
   245
<210> 2
   <211> 248
   <212> PRT
   <213> Bacillus subtilis
<400> 2
   Met Thr Gly Met Val Thr Glu Arg Arg Ser Val His Phe Ile Ala Glu
   1 5 10 15
   Ala Leu Thr Glu Asn Cys Arg Glu Ile Phe Glu Arg Arg Arg His Val
   20 25 30
   Leu Val Gly Ile Ser Pro Phe Asn Ser Arg Phe Ser Glu Asp Tyr Ile
   35 40 45
   Tyr Arg Leu Ile Gly Trp Ala Lys Ala Gln Phe Lys Ser Val Ser Val
   50 55 60
   Leu Leu Ala Gly His Glu Ala Ala Asn Leu Leu Glu Ala Leu Gly Thr
   65 70 75 80
   Pro Arg Gly Lys Ala Glu Arg Lys Val Arg Lys Glu Val Ser Arg Asn
   85 90 95
   Arg Arg Phe Ala Glu Arg Ala Leu Val Ala His Gly Gly Asp Pro Lys
   100 105 110
   Ala Ile His Thr Phe Ser Asp Phe Ile Asp Asn Lys Ala Tyr Gln Leu
   115 120 125
   Leu Arg Gln Glu Val Glu His Ala Phe Phe Glu Gln Pro His Phe Arg
   130 135 140
   His Ala Cys Leu Asp Met Ser Arg Glu Ala Ile Ile Gly Arg Ala Arg
   145 150 155 160
   Gly Val Ser Leu Met Met Glu Glu Val Ser Glu Asp Met Leu Asn Leu
   165 170 175
   Ala Val Glu Tyr Val Ile Ala Glu Leu Pro Phe Phe Ile Gly Ala Pro
   180 185 190
   Asp Ile Leu Glu Val Glu Glu Thr Leu Leu Ala Tyr His Arg Pro Trp
   195 200 205
   Lys Leu Gly Glu Lys Ile Ser Asn His Glu Phe Ser Ile Cys Met Arg
   210 215 220
   Pro Asn Gln Gly Tyr Leu Ile Val Gln Glu Met Ala Gln Met Leu Ser
   225 230 235 240
   Glu Lys Arg Ile Thr Ser Glu Gly
   245
<210> 3
   <211> 705
   <212> DNA
   <213> Photorhabdus luminescens subsp. laumondii
<220>
   <221> CDS
   <222> (1)..(705)
<400> 3
   atg ctg cac gag aat tca cca tca ttt act gtc caa ggt gaa acc tct          48
   Met Leu His Glu Asn Ser Pro Ser Phe Thr Val Gln Gly Glu Thr Ser
   1               5                   10                  15
   cgt tgt gac caa att att caa aaa ggt gat cac gcg cta ata ggg ata          96
   Arg Cys Asp Gln Ile Ile Gln Lys Gly Asp His Ala Leu Ile Gly Ile
               20                  25                  30
   agc ccc ttt aac tcg cgt ttt tca aaa gac tat gta gtg gac ctt att         144
   Ser Pro Phe Asn Ser Arg Phe Ser Lys Asp Tyr Val Val Asp Leu Ile
           35                  40                  45
   cag tgg tca agt cat tat ttc cga caa gtc gac ata tta tta cct tgt         192
   Gln Trp Ser Ser His Tyr Phe Arg Gln Val Asp Ile Leu Leu Pro Cys
       50                  55                  60
   gaa cgt gaa gct tca cgc ctt tta gtc gct agt gga att gat aat gtt         240
   Glu Arg Glu Ala Ser Arg Leu Leu Val Ala Ser Gly Ile Asp Asn Val
   65                  70                  75                  80
   aaa gct atc aaa aaa aca cat cgc gaa att aga cgt cat tta cgt aac         288
   Lys Ala Ile Lys Lys Thr His Arg Glu Ile Arg Arg His Leu Arg Asn
                   85                  90                  95
   ctt gat tat gtt att tcc aca gca aca ttg aaa agt aag caa atc aga         336
   Leu Asp Tyr Val Ile Ser Thr Ala Thr Leu Lys Ser Lys Gln Ile Arg
               100                 105                 110
   gtc atc caa ttt agt gac ttt tca cta aac cat gac tac caa tct ctt         384
   Val Ile Gln Phe Ser Asp Phe Ser Leu Asn His Asp Tyr Gln Ser Leu
           115                 120                 125
   aaa aca caa gtt gaa aac gcg ttt aat gaa tca gaa tct ttt aaa aaa         432
   Lys Thr Gln Val Glu Asn Ala Phe Asn Glu Ser Glu Ser Phe Lys Lys
       130                 135                 140
   agc tgt ctt gat atg tcc ttt caa gcc ata aaa ggg cga cta aaa ggt         480
   Ser Cys Leu Asp Met Ser Phe Gln Ala Ile Lys Gly Arg Leu Lys Gly
   145                 150                 155                 160
   act ggg caa tac ttt ggt caa att gac cta caa tta gta tat aaa gcg         528
   Thr Gly Gln Tyr Phe Gly Gln Ile Asp Leu Gln Leu Val Tyr Lys Ala
                   165                 170                 175
   ttg cca tat att ttc gct gaa att cct ttt tac ctc aat acc cct cga         576
   Leu Pro Tyr Ile Phe Ala Glu Ile Pro Phe Tyr Leu Asn Thr Pro Arg
               180                 185                 190
   tta ctt ggg gta aag tat tct acg tta ctt tat cac cgc cct tgg tca         624
   Leu Leu Gly Val Lys Tyr Ser Thr Leu Leu Tyr His Arg Pro Trp Ser
           195                 200                 205
   atc gga aaa ggg tta ttt aac ggt agt tat cct ata caa gta gca gat         672
   Ile Gly Lys Gly Leu Phe Asn Gly Ser Tyr Pro Ile Gln Val Ala Asp
       210                 215                 220
   aaa caa agt tac gga atc gtc act caa tta taa                             705
   Lys Gln Ser Tyr Gly Ile Val Thr Gln Leu
   225                 230
<210> 4
   <211> 234
   <212> PRT
   <213> Photorhabdus luminescens subsp. laumondii
<400> 4
   Met Leu His Glu Asn Ser Pro Ser Phe Thr Val Gln Gly Glu Thr Ser
   1               5                   10                  15
   Arg Cys Asp Gln Ile Ile Gln Lys Gly Asp His Ala Leu Ile Gly Ile
               20                  25                  30
   Ser Pro Phe Asn Ser Arg Phe Ser Lys Asp Tyr Val Val Asp Leu Ile
           35                  40                  45
   Gln Trp Ser Ser His Tyr Phe Arg Gln Val Asp Ile Leu Leu Pro Cys
       50                  55                  60
   Glu Arg Glu Ala Ser Arg Leu Leu Val Ala Ser Gly Ile Asp Asn Val
   65                  70                  75                  80
   Lys Ala Ile Lys Lys Thr His Arg Glu Ile Arg Arg His Leu Arg Asn
                   85                  90                  95
   Leu Asp Tyr Val Ile Ser Thr Ala Thr Leu Lys Ser Lys Gln Ile Arg
               100                 105                 110
   Val Ile Gln Phe Ser Asp Phe Ser Leu Asn His Asp Tyr Gln Ser Leu
           115                 120                 125
   Lys Thr Gln Val Glu Asn Ala Phe Asn Glu Ser Glu Ser Phe Lys Lys
       130                 135                 140
   Ser Cys Leu Asp Met Ser Phe Gln Ala Ile Lys Gly Arg Leu Lys Gly
   145                 150                 155                 160
   Thr Gly Gln Tyr Phe Gly Gln Ile Asp Leu Gln Leu Val Tyr Lys Ala
                   165                 170                 175
   Leu Pro Tyr Ile Phe Ala Glu Ile Pro Phe Tyr Leu Asn Thr Pro Arg
               180                 185                 190
   Leu Leu Gly Val Lys Tyr Ser Thr Leu Leu Tyr His Arg Pro Trp Ser
           195                 200                 205
   Ile Gly Lys Gly Leu Phe Asn Gly Ser Tyr Pro Ile Gln Val Ala Asp
       210                 215                 220
   Lys Gln Ser Tyr Gly Ile Val Thr Gln Leu
   225                 230
<210> 5
   <211> 720
   <212> DNA
   <213> Bacillus thuringiensis serovar israelensis
<220>
   <221> CDS
   <222> (1)..(720)
<400> 5
   atg acg aat gct ata gcg gta aga aat gta cga aag ttt agt tct caa      48
   Met Thr Asn Ala Ile Ala Val Arg Asn Val Arg Lys Phe Ser Ser Gln
   1               5                   10                  15
   ccc tta tct act aat tgt gct gaa ata tta aaa cgt agt aag cat gca      96
   Pro Leu Ser Thr Asn Cys Ala Glu Ile Leu Lys Arg Ser Lys His Ala
               20                  25                  30
   ata ata ggt att agt ccg ttt aat agt aga ttt tct gat gaa tat att     144
   Ile Ile Gly Ile Ser Pro Phe Asn Ser Arg Phe Ser Asp Glu Tyr Ile
           35                  40                  45
   aat aga ctc att gaa tgg gca tta cat act ttt gat gat gtt agt gtt     192
   Asn Arg Leu Ile Glu Trp Ala Leu His Thr Phe Asp Asp Val Ser Val
       50                  55                  60
   tta tta gct gga aaa gaa gct gca aat tta ctt gag gct cta gga aca     240
   Leu Leu Ala Gly Lys Glu Ala Ala Asn Leu Leu Glu Ala Leu Gly Thr
   65                  70                  75                  80
   cca aaa ggt aaa gcg gaa aga aaa gtt agg aaa gaa gta tct cga aat     288
   Pro Lys Gly Lys Ala Glu Arg Lys Val Arg Lys Glu Val Ser Arg Asn
                   85                  90                  95
   aga aga tca gct gaa aag gca ctt aaa gag cat ggt ggt aat gta aat     336
   Arg Arg Ser Ala Glu Lys Ala Leu Lys Glu His Gly Gly Asn Val Asn
               100                 105                 110
   gct atc cat act ttt tct gat ttt aat gac aac aat gca tat agc tgc     384
   Ala Ile His Thr Phe Ser Asp Phe Asn Asp Asn Asn Ala Tyr Ser Cys
           115                 120                 125
   atg agg gca gaa gca gaa cat att ttt tta agc gaa act gtt ttt cga     432
   Met Arg Ala Glu Ala Glu His Ile Phe Leu Ser Glu Thr Val Phe Arg
       130                 135                 140
   aat gct tgc tta gaa atg tca cat gca gcc att tta ggt agg gca agg     480
   Asn Ala Cys Leu Glu Met Ser His Ala Ala Ile Leu Gly Arg Ala Arg
   145                 150                 155                 160
   ggt act aat ata gat att gat caa ata tca aat gac atg cta aat atc     528
   Gly Thr Asn Ile Asp Ile Asp Gln Ile Ser Asn Asp Met Leu Asn Ile
                   165                 170                 175
   gca gta gaa tat gta att gca gaa ctc cca ttt ttc att ggt gga gct     576
   Ala Val Glu Tyr Val Ile Ala Glu Leu Pro Phe Phe Ile Gly Gly Ala
               180                 185                 190
   gaa att tta gga act caa gaa gct gta ctt att tat cat aaa cca tgg     624
   Glu Ile Leu Gly Thr Gln Glu Ala Val Leu Ile Tyr His Lys Pro Trp
           195                 200                 205
   gag ctt ggt gaa cag ata gtt aga aat gat ttt tct atc agg atg aaa     672
   Glu Leu Gly Glu Gln Ile Val Arg Asn Asp Phe Ser Ile Arg Met Lys
       210                 215                 220
   cca aat caa gga tat tta atg gta caa gac atg gaa aat tta tct taa     720
   Pro Asn Gln Gly Tyr Leu Met Val Gln Asp Met Glu Asn Leu Ser
   225                 230                 235
<210> 6
   <211> 239
   <212> PRT
   <213> Bacillus thuringiensis serovar israelensis
<400> 6
   Met Thr Asn Ala Ile Ala Val Arg Asn Val Arg Lys Phe Ser Ser Gln
   1 5 10 15
   Pro Leu Ser Thr Asn Cys Ala Glu Ile Leu Lys Arg Ser Lys His Ala
   20 25 30
   Ile Ile Gly Ile Ser Pro Phe Asn Ser Arg Phe Ser Asp Glu Tyr Ile
   35 40 45
   Asn Arg Leu Ile Glu Trp Ala Leu His Thr Phe Asp Asp Val Ser Val
   50 55 60
   Leu Leu Ala Gly Lys Glu Ala Ala Asn Leu Leu Glu Ala Leu Gly Thr
   65 70 75 80
   Pro Lys Gly Lys Ala Glu Arg Lys Val Arg Lys Glu Val Ser Arg Asn
   85 90 95
   Arg Arg Ser Ala Glu Lys Ala Leu Lys Glu His Gly Gly Asn Val Asn
   100 105 110
   Ala Ile His Thr Phe Ser Asp Phe Asn Asp Asn Asn Ala Tyr Ser Cys
   115 120 125
   Met Arg Ala Glu Ala Glu His Ile Phe Leu Ser Glu Thr Val Phe Arg
   130 135 140
   Asn Ala Cys Leu Glu Met Ser His Ala Ala Ile Leu Gly Arg Ala Arg
   145 150 155 160
   Gly Thr Asn Ile Asp Ile Asp Gln Ile Ser Asn Asp Met Leu Asn Ile
   165 170 175
   Ala Val Glu Tyr Val Ile Ala Glu Leu Pro Phe Phe Ile Gly Gly Ala
   180 185 190
   Glu Ile Leu Gly Thr Gln Glu Ala Val Leu Ile Tyr His Lys Pro Trp
   195 200 205
   Glu Leu Gly Glu Gln Ile Val Arg Asn Asp Phe Ser Ile Arg Met Lys
   210 215 220
   Pro Asn Gln Gly Tyr Leu Met Val Gln Asp Met Glu Asn Leu Ser
   225 230 235
<210> 7
   <211> 748
   <212> DNA
   <213> Bacillus licheniformis
<220>
   <221> CDS
   <222> (1)..(747)
<400> 7
   atg aca gag ctt ata atg gag agc aaa cac cag cta ttc aaa acc gaa      48
   Met Thr Glu Leu Ile Met Glu Ser Lys His Gln Leu Phe Lys Thr Glu
   1               5                   10                  15
   act ctt acc caa aac tgc aat gaa ata tta aaa cgc aga cgc cat gtt      96
   Thr Leu Thr Gln Asn Cys Asn Glu Ile Leu Lys Arg Arg Arg His Val
               20                  25                  30
   ctc gtc ggc atc agc ccg ttt aac agc cga ttt tcc gaa gat tat att     144
   Leu Val Gly Ile Ser Pro Phe Asn Ser Arg Phe Ser Glu Asp Tyr Ile
           35                  40                  45
   cat cgg ctt atc gcc tgg gcc gtc cgt gag ttt cag agt gta tcc gtg     192
   His Arg Leu Ile Ala Trp Ala Val Arg Glu Phe Gln Ser Val Ser Val
       50                  55                  60
   ctt ttg gcg gga aag gaa gct gcc aac ctt ctc gaa gcg ctc ggc acc     240
   Leu Leu Ala Gly Lys Glu Ala Ala Asn Leu Leu Glu Ala Leu Gly Thr
   65                  70                  75                  80
   cca cat ggg aag gcc gaa cgg aaa gtc agg aaa gaa gtc tcg cgg aac      288
   Pro His Gly Lys Ala Glu Arg Lys Val Arg Lys Glu Val Ser Arg Asn
                   85                  90                  95
   cgg aga ttc gct gaa aag gcg ttg gaa gcg cat ggc gga aat ccc gag      336
   Arg Arg Phe Ala Glu Lys Ala Leu Glu Ala His Gly Gly Asn Pro Glu
               100                 105                 110
   gac atc cat aca ttt tcc gat ttc gcg aac cag acc gca tac cgg aat      384
   Asp Ile His Thr Phe Ser Asp Phe Ala Asn Gln Thr Ala Tyr Arg Asn
           115                 120                 125
   ttg cgg atg gaa gtc gaa gct gcc ttt ttc gac cag acg cat ttt cgc      432
   Leu Arg Met Glu Val Glu Ala Ala Phe Phe Asp Gln Thr His Phe Arg
       130                 135                 140
   aat gcc tgc ctg gag atg tcg cat gcg gct atc ctc gga cgg gcc cgg      480
   Asn Ala Cys Leu Glu Met Ser His Ala Ala Ile Leu Gly Arg Ala Arg
   145                 150                 155                 160
   ggc act cgg atg gat gtc gtg gaa gtc agc gca gac atg ctg gag ctg      528
   Gly Thr Arg Met Asp Val Val Glu Val Ser Ala Asp Met Leu Glu Leu
                   165                 170                 175
   gct gtt gaa tac gtc atc gct gaa ctt ccg ttt ttc atc gcc gcc cct      576
   Ala Val Glu Tyr Val Ile Ala Glu Leu Pro Phe Phe Ile Ala Ala Pro
               180                 185                 190
   gat att tta ggc gtc gaa gag acg ctt ctt gct tat cac cgg cca tgg      624
   Asp Ile Leu Gly Val Glu Glu Thr Leu Leu Ala Tyr His Arg Pro Trp
           195                 200                 205
   aag ctc ggc gaa cag atc tcc cgt aat gaa ttt gcc gtc aaa atg cgg      672
   Lys Leu Gly Glu Gln Ile Ser Arg Asn Glu Phe Ala Val Lys Met Arg
       210                 215                 220
   ccg aat caa gga tat ctc atg gtt tcc gaa gcg gac gaa agg gtg gaa      720
   Pro Asn Gln Gly Tyr Leu Met Val Ser Glu Ala Asp Glu Arg Val Glu
   225                 230                 235                 240
   tct aaa agc atg cag gag gaa cga gta t                                748
   Ser Lys Ser Met Gln Glu Glu Arg Val
                   245
<210> 8
   <211> 249
   <212> PRT
   <213> Bacillus licheniformis
<400> 8
   Met Thr Glu Leu Ile Met Glu Ser Lys His Gln Leu Phe Lys Thr Glu
   1               5                   10                  15
   Thr Leu Thr Gln Asn Cys Asn Glu Ile Leu Lys Arg Arg Arg His Val
               20                  25                  30
   Leu Val Gly Ile Ser Pro Phe Asn Ser Arg Phe Ser Glu Asp Tyr Ile
           35                  40                  45
   His Arg Leu Ile Ala Trp Ala Val Arg Glu Phe Gln Ser Val Ser Val
       50                  55                  60
   Leu Leu Ala Gly Lys Glu Ala Ala Asn Leu Leu Glu Ala Leu Gly Thr
   65                  70                  75                  80
   Pro His Gly Lys Ala Glu Arg Lys Val Arg Lys Glu Val Ser Arg Asn
                   85                  90                  95
   Arg Arg Phe Ala Glu Lys Ala Leu Glu Ala His Gly Gly Asn Pro Glu
               100                 105                 110
   Asp Ile His Thr Phe Ser Asp Phe Ala Asn Gln Thr Ala Tyr Arg Asn
           115                 120                 125
   Leu Arg Met Glu Val Glu Ala Ala Phe Phe Asp Gln Thr His Phe Arg
       130                 135                 140
   Asn Ala Cys Leu Glu Met Ser His Ala Ala Ile Leu Gly Arg Ala Arg
   145                 150                 155                 160
   Gly Thr Arg Met Asp Val Val Glu Val Ser Ala Asp Met Leu Glu Leu
                   165                 170                 175
   Ala Val Glu Tyr Val Ile Ala Glu Leu Pro Phe Phe Ile Ala Ala Pro
               180                 185                 190
   Asp Ile Leu Gly Val Glu Glu Thr Leu Leu Ala Tyr His Arg Pro Trp
           195                 200                 205
   Lys Leu Gly Glu Gln Ile Ser Arg Asn Glu Phe Ala Val Lys Met Arg
       210                 215                 220
   Pro Asn Gln Gly Tyr Leu Met Val Ser Glu Ala Asp Glu Arg Val Glu
   225                 230                 235                 240
   Ser Lys Ser Met Gln Glu Glu Arg Val
                   245
<210> 9
   <211> 33
   <212> DNA
   <213> Artificial
<220>
   <223> primer A
<400> 9
   aagttttagg ggtgaatgag atgaccggaa tgg 33
<210> 10
   <211> 35
   <212> DNA
   <213> Artificial
<220>
   <223> primer B
<400> 10
   cgaagcttac tccccctatc atccttcaga tgtga 35
<210> 11
   <211> 42
   <212> DNA
   <213> Artificial
<220>
   <223> primer C
<400> 11
   ggcttcgaga atctttattt tcagggcacc ggaatggtaa cg 42
<210> 12
   <211> 52
   <212> DNA
   <213> Artificial
<220>
   <223> primer D
<400> 12
   ggggaccact ttgtacaaga aagctgggtc cttatccttc agatgtgatc cg 52
<210> 13
   <211> 47
   <212> DNA
   <213> Artificial
<220>
   <223> primer E
<400> 13
   ggggacaagt ttgtacaaaa aagcaggctt cgagaatctt tattttc 47
<210> 14
   <211> 20
   <212> DNA
   <213> Artificial
<220>
   <223> primer F
<400> 14
   atgaggcggc taatcttcta 20
<210> 15
   <211> 15
   <212> DNA
   <213> Artificial
<220>
   <223> primer M13 Forward
<400> 15
   gtaaacgacg gccag 15
<210> 16
   <211> 17
   <212> DNA
   <213> Artificial
<220>
<223> primer M13 Reverse
<400> 16
   caggaaacag ctatgac 17
<210> 17
   <211> 40
   <212> DNA
   <213> Artificial
<220>
   <223> primer A
<400> 17
   aacgaacaat caaatattat cagcccattc aacattgctg 40
<210> 18
   <211> 40
   <212> DNA
   <213> Artificial
<220>
   <223> primer B
<400> 18
   cgtattaact ttaaacgcag tgactatttc atcagactgt 40
<210> 19
   <211> 44
   <212> DNA
   <213> Artificial
<220>
   <223> primer C
<400> 19
   ggcttcgaga atctttattt tcagggcctg cacgagaatt cacc 44
<210> 20
   <211> 53
   <212> DNA
   <213> Artificial
<220>
   <223> primer D
<400> 20
   ggggaccact ttgtacaaga aagctgggtc cttataattg agtgacgatt ccg 53
<210> 21
   <211> 20
   <212> DNA
   <213> Artificial
<220>
   <223> primer F
<400> 21
   gccggtgata aagtaacgat 20
<210> 22
   <211> 47
   <212> DNA
   <213> Artificial
<220>
   <223> primer C
<400> 22
   ggcttcgaga atctttattt tcagggcacg aatgctatag cggtaag 47
<210> 23
   <211> 65
   <212> DNA
   <213> Artificial
<220>
   <223> primer D
<400> 23
   ggggggggga ccactttgta caagaaagct gggtccttaa gataaatttt ccatttcttg 60
   tacca 65
<210> 24
   <211> 43
   <212> DNA
   <213> Artificial
<220>
   <223> primer C
<400> 24
   ggcttcgaga atctttattt tcagggcaca gagcttataa tgg 43
<210> 25
   <211> 59
   <212> DNA
   <213> Artificial
<220>
   <223> primer D
<400> 25
   ggggggggga ccactttgta caagaaagct gggtccttat actcgttcct cctgcatgc 59
<210> 26
   <211> 5468
   <212> DNA
   <213> Artificial
<220>
   <223> expression vector pEXP-YvmCsub
<400> 26
   agatctcgat cccgcgaaat taatacgact cactataggg agaccacaac ggtttccctc     60
   tagaaataat tttgtttaac tttaagaagg agatatacat atgtcgtact accatcacca    120
   tcaccatcac ctcgaatcaa caagtttgta caaaaaagca ggcttcgaga atctttattt    180
   tcagggcacc ggaatggtaa cggaaagaag gtctgtgcat tttattgctg aggcattaac    240
   agaaaactgc agagaaatat ttgaacggcg caggcatgtt ttggtgggga tcagcccatt    300
   taacagcagg ttttcagagg attatattta cagattaatt ggatgggcga aagctcaatt 360
   taaaagcgtt tcagttttac ttgcagggca tgaggcggct aatcttctag aagcgcttgg 420
   aactccgaga ggaaaggctg aacgaaaagt aaggaaagag gtatcacgaa acaggagatt 480
   tgcagaaaga gcccttgtgg ctcatggcgg ggatccgaag gcgattcata cattttctga 540
   ttttatagat aacaaagcct accagctgtt gagacaagaa gttgaacatg cattttttga 600
   gcagcctcat tttcgacatg cttgtttgga catgtctcgt gaagcgataa tcgggcgtgc 660
   gcggggcgtc agtttgatga tggaagaagt cagtgaggat atgctgaatt tggctgtgga 720
   atatgtcata gctgagctgc cgttttttat cggagctccg gatattttag aggtggaaga 780
   gacactcctt gcttatcatc gtccgtggaa gctgggtgag aagatcagta accatgaatt 840
   ttctatttgt atgcggccga atcaagggta tctcattgta caggaaatgg cgcagatgct 900
   ttctgagaaa cggatcacat ctgaaggata aggacccagc tttcttgtac aaagtggttg 960
   attcgaggct gctaacaaag cccgaaagga agctgagttg gctgctgcca ccgctgagca 1020
   ataactagca taaccccttg gggcctctaa acgggtcttg aggggttttt tgctgaaagg 1080
   aggaactata tccggatatc cacaggacgg gtgtggtcgc catgatcgcg tagtcgatag 1140
   tggctccaag tagcgaagcg agcaggactg ggcggcggcc aaagcggtcg gacagtgctc 1200
   cgagaacggg tgcgcataga aattgcatca acgcatatag cgctagcagc acgccatagt 1260
   gactggcgat gctgtcggaa tggacgatat cccgcaagag gcccggcagt accggcataa 1320
   ccaagcctat gcctacagca tccagggtga cggtgccgag gatgacgatg agcgcattgt 1380
   tagatttcat acacggtgcc tgactgcgtt agcaatttaa ctgtgataaa ctaccgcatt 1440
   aaagcttatc gatgataagc tgtcaaacat gagaattctt gaagacgaaa gggcctcgtg 1500
   atacgcctat ttttataggt taatgtcatg ataataatgg tttcttagac gtcaggtggc 1560
   acttttcggg gaaatgtgcg cggaacccct atttgtttat ttttctaaat acattcaaat 1620
   atgtatccgc tcatgagaca ataaccctga taaatgcttc aataatattg aaaaaggaag 1680
   agtatgagta ttcaacattt ccgtgtcgcc cttattccct tttttgcggc attttgcctt 1740
   cctgtttttg ctcacccaga aacgctggtg aaagtaaaag atgctgaaga tcagttgggt 1800
   gcacgagtgg gttacatcga actggatctc aacagcggta agatccttga gagttttcgc 1860
   cccgaagaac gttttccaat gatgagcact tttaaagttc tgctatgtgg cgcggtatta 1920
   tcccgtgttg acgccgggca agagcaactc ggtcgccgca tacactattc tcagaatgac 1980
   ttggttgagt actcaccagt cacagaaaag catcttacgg atggcatgac agtaagagaa 2040
   ttatgcagtg ctgccataac catgagtgat aacactgcgg ccaacttact tctgacaacg 2100
   atcggaggac cgaaggagct aaccgctttt ttgcacaaca tgggggatca tgtaactcgc 2160
   cttgatcgtt gggaaccgga gctgaatgaa gccataccaa acgacgagcg tgacaccacg 2220
   atgcctgcag caatggcaac aacgttgcgc aaactattaa ctggcgaact acttactcta 2280
   gcttcccggc aacaattaat agactggatg gaggcggata aagttgcagg accacttctg 2340
   cgctcggccc ttccggctgg ctggtttatt gctgataaat ctggagccgg tgagcgtggg 2400
   tctcgcggta tcattgcagc actggggcca gatggtaagc cctcccgtat cgtagttatc 2460
   tacacgacgg ggagtcaggc aactatggat gaacgaaata gacagatcgc tgagataggt 2520
   gcctcactga ttaagcattg gtaactgtca gaccaagttt actcatatat actttagatt 2580
   gatttaaaac ttcattttta atttaaaagg atctaggtga agatcctttt tgataatctc 2640
   atgaccaaaa tcccttaacg tgagttttcg ttccactgag cgtcagaccc cgtagaaaag 2700
   atcaaaggat cttcttgaga tccttttttt ctgcgcgtaa tctgctgctt gcaaacaaaa 2760
   aaaccaccgc taccagcggt ggtttgtttg ccggatcaag agctaccaac tctttttccg 2820
   aaggtaactg gcttcagcag agcgcagata ccaaatactg tccttctagt gtagccgtag 2880
   ttaggccacc acttcaagaa ctctgtagca ccgcctacat acctcgctct gctaatcctg 2940
   ttaccagtgg ctgctgccag tggcgataag tcgtgtctta ccgggttgga ctcaagacga 3000
   tagttaccgg ataaggcgca gcggtcgggc tgaacggggg gttcgtgcac acagcccagc 3060
   ttggagcgaa cgacctacac cgaactgaga tacctacagc gtgagctatg agaaagcgcc 3120
   acgcttcccg aagggagaaa ggcggacagg tatccggtaa gcggcagggt cggaacagga 3180
   gagcgcacga gggagcttcc agggggaaac gcctggtatc tttatagtcc tgtcgggttt 3240
   cgccacctct gacttgagcg tcgatttttg tgatgctcgt caggggggcg gagcctatgg 3300
   aaaaacgcca gcaacgcggc ctttttacgg ttcctggcct tttgctggcc ttttgctcac 3360
   atgttctttc ctgcgttatc ccctgattct gtggataacc gtattaccgc ctttgagtga 3420
   gctgataccg ctcgccgcag ccgaacgacc gagcgcagcg agtcagtgag cgaggaagcg 3480
   gaagagcgcc tgatgcggta ttttctcctt acgcatctgt gcggtatttc acaccgcata 3540
   tatggtgcac tctcagtaca atctgctctg atgccgcata gttaagccag tatacactcc 3600
   gctatcgcta cgtgactggg tcatggctgc gccccgacac ccgccaacac ccgctgacgc 3660
   gccctgacgg gcttgtctgc tcccggcatc cgcttacaga caagctgtga ccgtctccgg 3720
   gagctgcatg tgtcagaggt tttcaccgtc atcaccgaaa cgcgcgaggc agctgcggta 3780
   aagctcatca gcgtggtcgt gaagcgattc acagatgtct gcctgttcat ccgcgtccag 3840
   ctcgttgagt ttctccagaa gcgttaatgt ctggcttctg ataaagcggg ccatgttaag 3900
   ggcggttttt tcctgtttgg tcactgatgc ctccgtgtaa gggggatttc tgttcatggg 3960
   ggtaatgata ccgatgaaac gagagaggat gctcacgata cgggttactg atgatgaaca 4020
   tgcccggtta ctggaacgtt gtgagggtaa acaactggcg gtatggatgc ggcgggacca 4080
   gagaaaaatc actcagggtc aatgccagcg cttcgttaat acagatgtag gtgttccaca 4140
   gggtagccag cagcatcctg cgatgcagat ccggaacata atggtgcagg gcgctgactt 4200
   ccgcgtttcc agactttacg aaacacggaa accgaagacc attcatgttg ttgctcaggt 4260
   cgcagacgtt ttgcagcagc agtcgcttca cgttcgctcg cgtatcggtg attcattctg 4320
   ctaaccagta aggcaacccc gccagcctag ccgggtcctc aacgacagga gcacgatcat 4380
   gcgcacccgt ggccaggacc caacgctgcc cgagatgcgc cgcgtgcggc tgctggagat 4440
   ggcggacgcg atggatatgt tctgccaagg gttggtttgc gcattcacag ttctccgcaa 4500
   gaattgattg gctccaattc ttggagtggt gaatccgtta gcgaggtgcc gccggcttcc 4560
   attcaggtcg aggtggcccg gctccatgca ccgcgacgca acgcggggag gcagacaagg 4620
   tatagggcgg cgcctacaat ccatgccaac ccgttccatg tgctcgccga ggcggcataa 4680
   atcgccgtga cgatcagcgg tccagtgatc gaagttaggc tggtaagagc cgcgagcgat 4740
   ccttgaagct gtccctgatg gtcgtcatct acctgcctgg acagcatggc ctgcaacgcg 4800
   ggcatcccga tgccgccgga agcgagaaga atcataatgg ggaaggccat ccagcctcgc 4860
   gtcgcgaacg ccagcaagac gtagcccagc gcgtcggccg ccatgccggc gataatggcc 4920
   tgcttctcgc cgaaacgttt ggtggcggga ccagtgacga aggcttgagc gagggcgtgc 4980
   aagattccga ataccgcaag cgacaggccg atcatcgtcg cgctccagcg aaagcggtcc 5040
   tcgccgaaaa tgacccagag cgctgccggc acctgtccta cgagttgcat gataaagaag 5100
   acagtcataa gtgcggcgac gatagtcatg ccccgcgccc accggaagga gctgactggg 5160
   ttgaaggctc tcaagggcat cggtcgatcg acgctctccc ttatgcgact cctgcattag 5220
   gaagcagccc agtagtaggt tgaggccgtt gagcaccgcc gccgcaagga atggtgcatg 5280
   caaggagatg gcgcccaaca gtcccccggc cacggggcct gccaccatac ccacgccgaa 5340
   acaagcgctc atgagcccga agtggcgagc ccgatcttcc ccatcggtga tgtcggcgat 5400
   ataggcgcca gcaaccgcac ctgtggcgcc ggtgatgccg gccacgatgc gtccggcgta 5460
   gaggatcg 5468
<210> 27
   <211> 5426
   <212> DNA
   <213> Artificial
<220>
   <223> expression vector pEXP-Plu0297
<400> 27
   agatctcgat cccgcgaaat taatacgact cactataggg agaccacaac ggtttccctc 60
   tagaaataat tttgtttaac tttaagaagg agatatacat atgtcgtact accatcacca 120
   tcaccatcac ctcgaatcaa caagtttgta caaaaaagca ggcttcgaga atctttattt 180
   tcagggcctg cacgagaatt caccatcatt tactgtccaa ggtgaaacct ctcgttgtga 240
   ccaaattatt caaaaaggtg atcacgcgct aatagggata agccccttta actcgcgttt 300
   ttcaaaagac tatgtagtgg accttattca gtggtcaagt cattatttcc gacaagtcga 360
   catattatta ccttgtgaac gtgaagcttc acgcctttta gtcgctagtg gaattgataa 420
   tgttaaagct atcaaaaaaa cacatcgcga aattagacgt catttacgta accttgatta 480
   tgttatttcc acagcaacat tgaaaagtaa gcaaatcaga gtcatccaat ttagtgactt 540
   ttcactaaac catgactacc aatctcttaa aacacaagtt gaaaacgcgt ttaatgaatc 600
   agaatctttt aaaaaaagct gtcttgatat gtcctttcaa gccataaaag ggcgactaaa 660
   aggtactggg caatactttg gtcaaattga cctacaatta gtatataaag cgttgccata 720
   tattttcgct gaaattcctt tttacctcaa tacccctcga ttacttgggg taaagtattc 780
   tacgttactt tatcaccgcc cttggtcaat cggaaaaggg ttatttaacg gtagttatcc 840
   tatacaagta gcagataaac aaagttacgg aatcgtcact caattataag gacccagctt 900
   tcttgtacaa agtggttgat tcgaggctgc taacaaagcc cgaaaggaag ctgagttggc 960
   tgctgccacc gctgagcaat aactagcata accccttggg gcctctaaac gggtcttgag 1020
   gggttttttg ctgaaaggag gaactatatc cggatatcca caggacgggt gtggtcgcca 1080
   tgatcgcgta gtcgatagtg gctccaagta gcgaagcgag caggactggg cggcggccaa 1140
   agcggtcgga cagtgctccg agaacgggtg cgcatagaaa ttgcatcaac gcatatagcg 1200
   ctagcagcac gccatagtga ctggcgatgc tgtcggaatg gacgatatcc cgcaagaggc 1260
   ccggcagtac cggcataacc aagcctatgc ctacagcatc cagggtgacg gtgccgagga 1320
   tgacgatgag cgcattgtta gatttcatac acggtgcctg actgcgttag caatttaact 1380
   gtgataaact accgcattaa agcttatcga tgataagctg tcaaacatga gaattcttga 1440
   agacgaaagg gcctcgtgat acgcctattt ttataggtta atgtcatgat aataatggtt 1500
   tcttagacgt caggtggcac ttttcgggga aatgtgcgcg gaacccctat ttgtttattt 1560
   ttctaaatac attcaaatat gtatccgctc atgagacaat aaccctgata aatgcttcaa 1620
   taatattgaa aaaggaagag tatgagtatt caacatttcc gtgtcgccct tattcccttt 1680
   tttgcggcat tttgccttcc tgtttttgct cacccagaaa cgctggtgaa agtaaaagat 1740
   gctgaagatc agttgggtgc acgagtgggt tacatcgaac tggatctcaa cagcggtaag 1800
   atccttgaga gttttcgccc cgaagaacgt tttccaatga tgagcacttt taaagttctg 1860
   ctatgtggcg cggtattatc ccgtgttgac gccgggcaag agcaactcgg tcgccgcata 1920
   cactattctc agaatgactt ggttgagtac tcaccagtca cagaaaagca tcttacggat 1980
   ggcatgacag taagagaatt atgcagtgct gccataacca tgagtgataa cactgcggcc 2040
   aacttacttc tgacaacgat cggaggaccg aaggagctaa ccgctttttt gcacaacatg 2100
   ggggatcatg taactcgcct tgatcgttgg gaaccggagc tgaatgaagc cataccaaac 2160
   gacgagcgtg acaccacgat gcctgcagca atggcaacaa cgttgcgcaa actattaact 2220
   ggcgaactac ttactctagc ttcccggcaa caattaatag actggatgga ggcggataaa 2280
   gttgcaggac cacttctgcg ctcggccctt ccggctggct ggtttattgc tgataaatct 2340
   ggagccggtg agcgtgggtc tcgcggtatc attgcagcac tggggccaga tggtaagccc 2400
   tcccgtatcg tagttatcta cacgacgggg agtcaggcaa ctatggatga acgaaataga 2460
   cagatcgctg agataggtgc ctcactgatt aagcattggt aactgtcaga ccaagtttac 2520
   tcatatatac tttagattga tttaaaactt catttttaat ttaaaaggat ctaggtgaag 2580
   atcctttttg ataatctcat gaccaaaatc ccttaacgtg agttttcgtt ccactgagcg 2640
   tcagaccccg tagaaaagat caaaggatct tcttgagatc ctttttttct gcgcgtaatc   2700
   tgctgcttgc aaacaaaaaa accaccgcta ccagcggtgg tttgtttgcc ggatcaagag   2760
   ctaccaactc tttttccgaa ggtaactggc ttcagcagag cgcagatacc aaatactgtc   2820
   cttctagtgt agccgtagtt aggccaccac ttcaagaact ctgtagcacc gcctacatac   2880
   ctcgctctgc taatcctgtt accagtggct gctgccagtg gcgataagtc gtgtcttacc   2940
   gggttggact caagacgata gttaccggat aaggcgcagc ggtcgggctg aacggggggt   3000
   tcgtgcacac agcccagctt ggagcgaacg acctacaccg aactgagata cctacagcgt   3060
   gagctatgag aaagcgccac gcttcccgaa gggagaaagg cggacaggta tccggtaagc   3120
   ggcagggtcg gaacaggaga gcgcacgagg gagcttccag ggggaaacgc ctggtatctt   3180
   tatagtcctg tcgggtttcg ccacctctga cttgagcgtc gatttttgtg atgctcgtca   3240
   ggggggcgga gcctatggaa aaacgccagc aacgcggcct ttttacggtt cctggccttt   3300
   tgctggcctt ttgctcacat gttctttcct gcgttatccc ctgattctgt ggataaccgt   3360
   attaccgcct ttgagtgagc tgataccgct cgccgcagcc gaacgaccga gcgcagcgag   3420
   tcagtgagcg aggaagcgga agagcgcctg atgcggtatt ttctccttac gcatctgtgc   3480
   ggtatttcac accgcatata tggtgcactc tcagtacaat ctgctctgat gccgcatagt   3540
   taagccagta tacactccgc tatcgctacg tgactgggtc atggctgcgc cccgacaccc   3600
   gccaacaccc gctgacgcgc cctgacgggc ttgtctgctc ccggcatccg cttacagaca   3660
   agctgtgacc gtctccggga gctgcatgtg tcagaggttt tcaccgtcat caccgaaacg   3720
   cgcgaggcag ctgcggtaaa gctcatcagc gtggtcgtga agcgattcac agatgtctgc   3780
   ctgttcatcc gcgtccagct cgttgagttt ctccagaagc gttaatgtct ggcttctgat   3840
   aaagcgggcc atgttaaggg cggttttttc ctgtttggtc actgatgcct ccgtgtaagg   3900
   gggatttctg ttcatggggg taatgatacc gatgaaacga gagaggatgc tcacgatacg   3960
   ggttactgat gatgaacatg cccggttact ggaacgttgt gagggtaaac aactggcggt   4020
   atggatgcgg cgggaccaga gaaaaatcac tcagggtcaa tgccagcgct tcgttaatac   4080
   agatgtaggt gttccacagg gtagccagca gcatcctgcg atgcagatcc ggaacataat   4140
   ggtgcagggc gctgacttcc gcgtttccag actttacgaa acacggaaac cgaagaccat   4200
   tcatgttgtt gctcaggtcg cagacgtttt gcagcagcag tcgcttcacg ttcgctcgcg   4260
   tatcggtgat tcattctgct aaccagtaag gcaaccccgc cagcctagcc gggtcctcaa   4320
   cgacaggagc acgatcatgc gcacccgtgg ccaggaccca acgctgcccg agatgcgccg   4380
   cgtgcggctg ctggagatgg cggacgcgat ggatatgttc tgccaagggt tggtttgcgc   4440
   attcacagtt ctccgcaaga attgattggc tccaattctt ggagtggtga atccgttagc   4500
   gaggtgccgc cggcttccat tcaggtcgag gtggcccggc tccatgcacc gcgacgcaac   4560
   gcggggaggc agacaaggta tagggcggcg cctacaatcc atgccaaccc gttccatgtg   4620
   ctcgccgagg cggcataaat cgccgtgacg atcagcggtc cagtgatcga agttaggctg   4680
   gtaagagccg cgagcgatcc ttgaagctgt ccctgatggt cgtcatctac ctgcctggac 4740
   agcatggcct gcaacgcggg catcccgatg ccgccggaag cgagaagaat cataatgggg 4800
   aaggccatcc agcctcgcgt cgcgaacgcc agcaagacgt agcccagcgc gtcggccgcc 4860
   atgccggcga taatggcctg cttctcgccg aaacgtttgg tggcgggacc agtgacgaag 4920
   gcttgagcga gggcgtgcaa gattccgaat accgcaagcg acaggccgat catcgtcgcg 4980
   ctccagcgaa agcggtcctc gccgaaaatg acccagagcg ctgccggcac ctgtcctacg 5040
   agttgcatga taaagaagac agtcataagt gcggcgacga tagtcatgcc ccgcgcccac 5100
   cggaaggagc tgactgggtt gaaggctctc aagggcatcg gtcgatcgac gctctccctt 5160
   atgcgactcc tgcattagga agcagcccag tagtaggttg aggccgttga gcaccgccgc 5220
   cgcaaggaat ggtgcatgca aggagatggc gcccaacagt cccccggcca cggggcctgc 5280
   caccataccc acgccgaaac aagcgctcat gagcccgaag tggcgagccc gatcttcccc 5340
   atcggtgatg tcggcgatat aggcgccagc aaccgcacct gtggcgccgg tgatgccggc 5400
   cacgatgcgt ccggcgtaga ggatcg 5426
<210> 28
   <211> 5441
   <212> DNA
   <213> Artificial
<220>
   <223> expression vector pEXP-YvmCthu
<400> 28
   agatctcgat cccgcgaaat taatacgact cactataggg agaccacaac ggtttccctc 60
   tagaaataat tttgtttaac tttaagaagg agatatacat atgtcgtact accatcacca 120
   tcaccatcac ctcgaatcaa caagtttgta caaaaaagca ggcttcgaga atctttattt 180
   tcagggcacg aatgctatag cggtaagaaa tgtacgaaag tttagttctc aacccttatc 240
   tactaattgt gctgaaatat taaaacgtag taagcatgca ataataggta ttagtccgtt 300
   taatagtaga ttttctgatg aatatattaa tagactcatt gaatgggcat tacatacttt 360
   tgatgatgtt agtgttttat tagctggaaa agaagctgca aatttacttg aggctctagg 420
   aacaccaaaa ggtaaagcgg aaagaaaagt taggaaagaa gtatctcgaa atagaagatc 480
   agctgaaaag gcacttaaag agcatggtgg taatgtaaat gctatccata ctttttctga 540
   ttttaatgac aacaatgcat atagctgcat gagggcagaa gcagaacata tttttttaag 600
   cgaaactgtt tttcgaaatg cttgcttaga aatgtcacat gcagccattt taggtagggc 660
   aaggggtact aatatagata ttgatcaaat atcaaatgac atgctaaata tcgcagtaga 720
   atatgtaatt gcagaactcc catttttcat tggtggagct gaaattttag gaactcaaga 780
   agctgtactt atttatcata aaccatggga gcttggtgaa cagatagtta gaaatgattt 840
   ttctatcagg atgaaaccaa atcaaggata tttaatggta caagacatgg aaaatttatc 900
   ttaaggaccc agctttcttg tacaaagtgg ttgattcgag gctgctaaca aagcccgaaa 960
   ggaagctgag ttggctgctg ccaccgctga gcaataacta gcataacccc ttggggcctc 1020
   taaacgggtc ttgaggggtt ttttgctgaa aggaggaact atatccggat atccacagga 1080
   cgggtgtggt cgccatgatc gcgtagtcga tagtggctcc aagtagcgaa gcgagcagga 1140
   ctgggcggcg gccaaagcgg tcggacagtg ctccgagaac gggtgcgcat agaaattgca 1200
   tcaacgcata tagcgctagc agcacgccat agtgactggc gatgctgtcg gaatggacga 1260
   tatcccgcaa gaggcccggc agtaccggca taaccaagcc tatgcctaca gcatccaggg 1320
   tgacggtgcc gaggatgacg atgagcgcat tgttagattt catacacggt gcctgactgc 1380
   gttagcaatt taactgtgat aaactaccgc attaaagctt atcgatgata agctgtcaaa 1440
   catgagaatt cttgaagacg aaagggcctc gtgatacgcc tatttttata ggttaatgtc 1500
   atgataataa tggtttctta gacgtcaggt ggcacttttc ggggaaatgt gcgcggaacc 1560
   cctatttgtt tatttttcta aatacattca aatatgtatc cgctcatgag acaataaccc 1620
   tgataaatgc ttcaataata ttgaaaaagg aagagtatga gtattcaaca tttccgtgtc 1680
   gcccttattc ccttttttgc ggcattttgc cttcctgttt ttgctcaccc agaaacgctg 1740
   gtgaaagtaa aagatgctga agatcagttg ggtgcacgag tgggttacat cgaactggat 1800
   ctcaacagcg gtaagatcct tgagagtttt cgccccgaag aacgttttcc aatgatgagc 1860
   acttttaaag ttctgctatg tggcgcggta ttatcccgtg ttgacgccgg gcaagagcaa 1920
   ctcggtcgcc gcatacacta ttctcagaat gacttggttg agtactcacc agtcacagaa 1980
   aagcatctta cggatggcat gacagtaaga gaattatgca gtgctgccat aaccatgagt 2040
   gataacactg cggccaactt acttctgaca acgatcggag gaccgaagga gctaaccgct 2100
   tttttgcaca acatggggga tcatgtaact cgccttgatc gttgggaacc ggagctgaat 2160
   gaagccatac caaacgacga gcgtgacacc acgatgcctg cagcaatggc aacaacgttg 2220
   cgcaaactat taactggcga actacttact ctagcttccc ggcaacaatt aatagactgg 2280
   atggaggcgg ataaagttgc aggaccactt ctgcgctcgg cccttccggc tggctggttt 2340
   attgctgata aatctggagc cggtgagcgt gggtctcgcg gtatcattgc agcactgggg 2400
   ccagatggta agccctcccg tatcgtagtt atctacacga cggggagtca ggcaactatg 2460
   gatgaacgaa atagacagat cgctgagata ggtgcctcac tgattaagca ttggtaactg 2520
   tcagaccaag tttactcata tatactttag attgatttaa aacttcattt ttaatttaaa 2580
   aggatctagg tgaagatcct ttttgataat ctcatgacca aaatccctta acgtgagttt 2640
   tcgttccact gagcgtcaga ccccgtagaa aagatcaaag gatcttcttg agatcctttt 2700
   tttctgcgcg taatctgctg cttgcaaaca aaaaaaccac cgctaccagc ggtggtttgt 2760
   ttgccggatc aagagctacc aactcttttt ccgaaggtaa ctggcttcag cagagcgcag 2820
   ataccaaata ctgtccttct agtgtagccg tagttaggcc accacttcaa gaactctgta 2880
   gcaccgccta catacctcgc tctgctaatc ctgttaccag tggctgctgc cagtggcgat 2940
   aagtcgtgtc ttaccgggtt ggactcaaga cgatagttac cggataaggc gcagcggtcg 3000
   ggctgaacgg ggggttcgtg cacacagccc agcttggagc gaacgaccta caccgaactg 3060
   agatacctac agcgtgagct atgagaaagc gccacgcttc ccgaagggag aaaggcggac 3120
   aggtatccgg taagcggcag ggtcggaaca ggagagcgca cgagggagct tccaggggga 3180
   aacgcctggt atctttatag tcctgtcggg tttcgccacc tctgacttga gcgtcgattt 3240
   ttgtgatgct cgtcaggggg gcggagccta tggaaaaacg ccagcaacgc ggccttttta 3300
   cggttcctgg ccttttgctg gccttttgct cacatgttct ttcctgcgtt atcccctgat 3360
   tctgtggata accgtattac cgcctttgag tgagctgata ccgctcgccg cagccgaacg 3420
   accgagcgca gcgagtcagt gagcgaggaa gcggaagagc gcctgatgcg gtattttctc 3480
   cttacgcatc tgtgcggtat ttcacaccgc atatatggtg cactctcagt acaatctgct 3540
   ctgatgccgc atagttaagc cagtatacac tccgctatcg ctacgtgact gggtcatggc 3600
   tgcgccccga cacccgccaa cacccgctga cgcgccctga cgggcttgtc tgctcccggc 3660
   atccgcttac agacaagctg tgaccgtctc cgggagctgc atgtgtcaga ggttttcacc 3720
   gtcatcaccg aaacgcgcga ggcagctgcg gtaaagctca tcagcgtggt cgtgaagcga 3780
   ttcacagatg tctgcctgtt catccgcgtc cagctcgttg agtttctcca gaagcgttaa 3840
   tgtctggctt ctgataaagc gggccatgtt aagggcggtt ttttcctgtt tggtcactga 3900
   tgcctccgtg taagggggat ttctgttcat gggggtaatg ataccgatga aacgagagag 3960
   gatgctcacg atacgggtta ctgatgatga acatgcccgg ttactggaac gttgtgaggg 4020
   taaacaactg gcggtatgga tgcggcggga ccagagaaaa atcactcagg gtcaatgcca 4080
   gcgcttcgtt aatacagatg taggtgttcc acagggtagc cagcagcatc ctgcgatgca 4140
   gatccggaac ataatggtgc agggcgctga cttccgcgtt tccagacttt acgaaacacg 4200
   gaaaccgaag accattcatg ttgttgctca ggtcgcagac gttttgcagc agcagtcgct 4260
   tcacgttcgc tcgcgtatcg gtgattcatt ctgctaacca gtaaggcaac cccgccagcc 4320
   tagccgggtc ctcaacgaca ggagcacgat catgcgcacc cgtggccagg acccaacgct 4380
   gcccgagatg cgccgcgtgc ggctgctgga gatggcggac gcgatggata tgttctgcca 4440
   agggttggtt tgcgcattca cagttctccg caagaattga ttggctccaa ttcttggagt 4500
   ggtgaatccg ttagcgaggt gccgccggct tccattcagg tcgaggtggc ccggctccat 4560
   gcaccgcgac gcaacgcggg gaggcagaca aggtataggg cggcgcctac aatccatgcc 4620
   aacccgttcc atgtgctcgc cgaggcggca taaatcgccg tgacgatcag cggtccagtg 4680
   atcgaagtta ggctggtaag agccgcgagc gatccttgaa gctgtccctg atggtcgtca 4740
   tctacctgcc tggacagcat ggcctgcaac gcgggcatcc cgatgccgcc ggaagcgaga 4800
   agaatcataa tggggaaggc catccagcct cgcgtcgcga acgccagcaa gacgtagccc 4860
   agcgcgtcgg ccgccatgcc ggcgataatg gcctgcttct cgccgaaacg tttggtggcg 4920
   ggaccagtga cgaaggcttg agcgagggcg tgcaagattc cgaataccgc aagcgacagg 4980
   ccgatcatcg tcgcgctcca gcgaaagcgg tcctcgccga aaatgaccca gagcgctgcc 5040
   ggcacctgtc ctacgagttg catgataaag aagacagtca taagtgcggc gacgatagtc 5100
   atgccccgcg cccaccggaa ggagctgact gggttgaagg ctctcaaggg catcggtcga 5160
   tcgacgctct cccttatgcg actcctgcat taggaagcag cccagtagta ggttgaggcc 5220
   gttgagcacc gccgccgcaa ggaatggtgc atgcaaggag atggcgccca acagtccccc 5280
   ggccacgggg cctgccacca tacccacgcc gaaacaagcg ctcatgagcc cgaagtggcg 5340
   agcccgatct tccccatcgg tgatgtcggc gatataggcg ccagcaaccg cacctgtggc 5400
   gccggtgatg ccggccacga tgcgtccggc gtagaggatc g 5441
<210> 29
   <211> 5471
   <212> DNA
   <213> Artificial
<220>
   <223> expression vector pEXP-YvmClic
<400> 29
   agatctcgat cccgcgaaat taatacgact cactataggg agaccacaac ggtttccctc      60
   tagaaataat tttgtttaac tttaagaagg agatatacat atgtcgtact accatcacca     120
   tcaccatcac ctcgaatcaa caagtttgta caaaaaagca ggcttcgaga atctttattt     180
   tcagggcaca gagcttataa tggagagcaa acaccagcta ttcaaaaccg aaactcttac     240
   ccaaaactgc aatgaaatat taaaacgcag acgccatgtt ctcgtcggca tcagcccgtt     300
   taacagccga ttttccgaag attatattca tcggcttatc gcctgggccg tccgtgagtt     360
   tcagagtgta tccgtgcttt tggcgggaaa ggaagctgcc aaccttctcg aagcgctcgg     420
   caccccacat gggaaggccg aacggaaagt caggaaagaa gtctcgcgga accggagatt     480
   cgctgaaaag gcgttggaag cgcatggcgg aaatcccgag gacatccata cattttccga     540
   tttcgcgaac cagaccgcat accggaattt gcggatggaa gtcgaagctg cctttttcga    600
   ccagacgcat tttcgcaatg cctgcctgga gatgtcgcat gcggctatcc tcggacgggc     660
   ccggggcact cggatggatg tcgtggaagt cagcgcagac atgctggagc tggctgttga     720
   atacgtcatc gctgaacttc cgtttttcat cgccgcccct gatattttag gcgtcgaaga     780
   gacgcttctt gcttatcacc ggccatggaa gctcggcgaa cagatctccc gtaatgaatt     840
   tgccgtcaaa atgcggccga atcaaggata tctcatggtt tccgaagcgg acgaaagggt     900
   ggaatctaaa agcatgcagg aggaacgagt ataaggaccc agctttcttg tacaaagtgg     960
   ttgattcgag gctgctaaca aagcccgaaa ggaagctgag ttggctgctg ccaccgctga    1020
   gcaataacta gcataacccc ttggggcctc taaacgggtc ttgaggggtt ttttgctgaa    1080
   aggaggaact atatccggat atccacagga cgggtgtggt cgccatgatc gcgtagtcga    1140
   tagtggctcc aagtagcgaa gcgagcagga ctgggcggcg gccaaagcgg tcggacagtg    1200
   ctccgagaac gggtgcgcat agaaattgca tcaacgcata tagcgctagc agcacgccat    1260
   agtgactggc gatgctgtcg gaatggacga tatcccgcaa gaggcccggc agtaccggca    1320
   taaccaagcc tatgcctaca gcatccaggg tgacggtgcc gaggatgacg atgagcgcat 1380
   tgttagattt catacacggt gcctgactgc gttagcaatt taactgtgat aaactaccgc 1440
   attaaagctt atcgatgata agctgtcaaa catgagaatt cttgaagacg aaagggcctc 1500
   gtgatacgcc tatttttata ggttaatgtc atgataataa tggtttctta gacgtcaggt 1560
   ggcacttttc ggggaaatgt gcgcggaacc cctatttgtt tatttttcta aatacattca 1620
   aatatgtatc cgctcatgag acaataaccc tgataaatgc ttcaataata ttgaaaaagg 1680
   aagagtatga gtattcaaca tttccgtgtc gcccttattc ccttttttgc ggcattttgc 1740
   cttcctgttt ttgctcaccc agaaacgctg gtgaaagtaa aagatgctga agatcagttg 1800
   ggtgcacgag tgggttacat cgaactggat ctcaacagcg gtaagatcct tgagagtttt 1860
   cgccccgaag aacgttttcc aatgatgagc acttttaaag ttctgctatg tggcgcggta 1920
   ttatcccgtg ttgacgccgg gcaagagcaa ctcggtcgcc gcatacacta ttctcagaat 1980
   gacttggttg agtactcacc agtcacagaa aagcatctta cggatggcat gacagtaaga 2040
   gaattatgca gtgctgccat aaccatgagt gataacactg cggccaactt acttctgaca 2100
   acgatcggag gaccgaagga gctaaccgct tttttgcaca acatggggga tcatgtaact 2160
   cgccttgatc gttgggaacc ggagctgaat gaagccatac caaacgacga gcgtgacacc 2220
   acgatgcctg cagcaatggc aacaacgttg cgcaaactat taactggcga actacttact 2280
   ctagcttccc ggcaacaatt aatagactgg atggaggcgg ataaagttgc aggaccactt 2340
   ctgcgctcgg cccttccggc tggctggttt attgctgata aatctggagc cggtgagcgt 2400
   gggtctcgcg gtatcattgc agcactgggg ccagatggta agccctcccg tatcgtagtt 2460
   atctacacga cggggagtca ggcaactatg gatgaacgaa atagacagat cgctgagata 2520
   ggtgcctcac tgattaagca ttggtaactg tcagaccaag tttactcata tatactttag 2580
   attgatttaa aacttcattt ttaatttaaa aggatctagg tgaagatcct ttttgataat 2640
   ctcatgacca aaatccctta acgtgagttt tcgttccact gagcgtcaga ccccgtagaa 2700
   aagatcaaag gatcttcttg agatcctttt tttctgcgcg taatctgctg cttgcaaaca 2760
   aaaaaaccac cgctaccagc ggtggtttgt ttgccggatc aagagctacc aactcttttt 2820
   ccgaaggtaa ctggcttcag cagagcgcag ataccaaata ctgtccttct agtgtagccg 2880
   tagttaggcc accacttcaa gaactctgta gcaccgccta catacctcgc tctgctaatc 2940
   ctgttaccag tggctgctgc cagtggcgat aagtcgtgtc ttaccgggtt ggactcaaga 3000
   cgatagttac cggataaggc gcagcggtcg ggctgaacgg ggggttcgtg cacacagccc 3060
   agcttggagc gaacgaccta caccgaactg agatacctac agcgtgagct atgagaaagc 3120
   gccacgcttc ccgaagggag aaaggcggac aggtatccgg taagcggcag ggtcggaaca 3180
   ggagagcgca cgagggagct tccaggggga aacgcctggt atctttatag tcctgtcggg 3240
   tttcgccacc tctgacttga gcgtcgattt ttgtgatgct cgtcaggggg gcggagccta 3300
   tggaaaaacg ccagcaacgc ggccttttta cggttcctgg ccttttgctg gccttttgct 3360
   cacatgttct ttcctgcgtt atcccctgat tctgtggata accgtattac cgcctttgag 3420
   tgagctgata ccgctcgccg cagccgaacg accgagcgca gcgagtcagt gagcgaggaa 3480
   gcggaagagc gcctgatgcg gtattttctc cttacgcatc tgtgcggtat ttcacaccgc 3540
   atatatggtg cactctcagt acaatctgct ctgatgccgc atagttaagc cagtatacac 3600
   tccgctatcg ctacgtgact gggtcatggc tgcgccccga cacccgccaa cacccgctga 3660
   cgcgccctga cgggcttgtc tgctcccggc atccgcttac agacaagctg tgaccgtctc 3720
   cgggagctgc atgtgtcaga ggttttcacc gtcatcaccg aaacgcgcga ggcagctgcg 3780
   gtaaagctca tcagcgtggt cgtgaagcga ttcacagatg tctgcctgtt catccgcgtc 3840
   cagctcgttg agtttctcca gaagcgttaa tgtctggctt ctgataaagc gggccatgtt 3900
   aagggcggtt ttttcctgtt tggtcactga tgcctccgtg taagggggat ttctgttcat 3960
   gggggtaatg ataccgatga aacgagagag gatgctcacg atacgggtta ctgatgatga 4020
   acatgcccgg ttactggaac gttgtgaggg taaacaactg gcggtatgga tgcggcggga 4080
   ccagagaaaa atcactcagg gtcaatgcca gcgcttcgtt aatacagatg taggtgttcc 4140
   acagggtagc cagcagcatc ctgcgatgca gatccggaac ataatggtgc agggcgctga 4200
   cttccgcgtt tccagacttt acgaaacacg gaaaccgaag accattcatg ttgttgctca 4260
   ggtcgcagac gttttgcagc agcagtcgct tcacgttcgc tcgcgtatcg gtgattcatt 4320
   ctgctaacca gtaaggcaac cccgccagcc tagccgggtc ctcaacgaca ggagcacgat 4380
   catgcgcacc cgtggccagg acccaacgct gcccgagatg cgccgcgtgc ggctgctgga 4440
   gatggcggac gcgatggata tgttctgcca agggttggtt tgcgcattca cagttctccg 4500
   caagaattga ttggctccaa ttcttggagt ggtgaatccg ttagcgaggt gccgccggct 4560
   tccattcagg tcgaggtggc ccggctccat gcaccgcgac gcaacgcggg gaggcagaca 4620
   aggtataggg cggcgcctac aatccatgcc aacccgttcc atgtgctcgc cgaggcggca 4680
   taaatcgccg tgacgatcag cggtccagtg atcgaagtta ggctggtaag agccgcgagc 4740
   gatccttgaa gctgtccctg atggtcgtca tctacctgcc tggacagcat ggcctgcaac 4800
   gcgggcatcc cgatgccgcc ggaagcgaga agaatcataa tggggaaggc catccagcct 4860
   cgcgtcgcga acgccagcaa gacgtagccc agcgcgtcgg ccgccatgcc ggcgataatg 4920
   gcctgcttct cgccgaaacg tttggtggcg ggaccagtga cgaaggcttg agcgagggcg 4980
   tgcaagattc cgaataccgc aagcgacagg ccgatcatcg tcgcgctcca gcgaaagcgg 5040
   tcctcgccga aaatgaccca gagcgctgcc ggcacctgtc ctacgagttg catgataaag 5100
   aagacagtca taagtgcggc gacgatagtc atgccccgcg cccaccggaa ggagctgact 5160
   gggttgaagg ctctcaaggg catcggtcga tcgacgctct cccttatgcg actcctgcat 5220
   taggaagcag cccagtagta ggttgaggcc gttgagcacc gccgccgcaa ggaatggtgc 5280
   atgcaaggag atggcgccca acagtccccc ggccacgggg cctgccacca tacccacgcc 5340
   gaaacaagcg ctcatgagcc cgaagtggcg agcccgatct tccccatcgg tgatgtcggc 5400
   gatataggcg ccagcaaccg cacctgtggc gccggtgatg ccggccacga tgcgtccggc    5460
   gtagaggatc g                                                         5471

## Description

The present invention relates to isolated, natural or synthetic polynucleotides and to the polypeptides encoded by said polynucleotides, that are involved in the synthesis of cyclodipeptides, to the recombinant vectors comprising said polynucleotides or any substantially homologous polynucleotides, to the host cell modified with said polynucleotides or said recombinant vectors and also to methods for *in vitro* and *in vivo* synthesizing cyclo(Leu-Leu) cyclodipeptide and its derivatives.

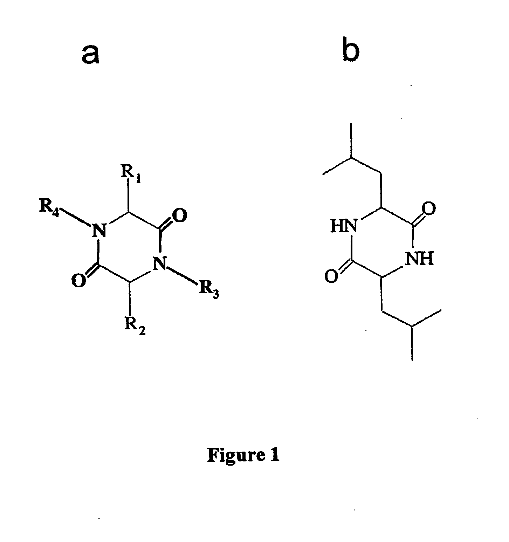

For the purposes of the present invention, the term "diketopiperazine derivatives" or "DKP" or "2,5-DKP" or "cyclic dipeptides" or "cyclodipeptides" or "cyclic diamino acids" is intended to mean molecules having a diketopiperazine (piperazine-2,5-dione or 2,5-dioxopiperazine) ring. In the particular case of α,β-dehydrogenated cyclodipeptide derivatives, the substituent groups R1 and R2 are α,β-unsaturated amino acyl side chains (Figure 1). Such derivatives are hereafter referred to as "Δ" derivatives.

The DKP derivatives constitute a growing family of compounds that are naturally produced by many organisms such as bacteria, yeast, filamentous fungi and lichens. Others have also been isolated from marine organisms, such as sponges and starfish. An example of these derivatives: cyclo(L-His-L-Pro), has been shown to be present in mammals.

The DKP derivatives display a very wide diversity of structures ranging from simple cyclodipeptides to much more complex structures. The simple cyclodipeptides constitute only a small fraction of the DKP derivatives, the majority of which have more complex structures in which the main ring and/or the side chains comprise many modifications: introduction of carbon-based, hydroxyl, nitro, epoxy, acetyl or methoxy groups, and also the formation of disulfide bridges or of heterocycles. The formation of a double bond between two carbons is also quite widespread. Certain derivatives, of marine origin, incorporate halogen atoms.

Useful biological properties have already been demonstrated for some of the DKP derivatives. Bicyclomycin (Bicozamine™) is an antibacterial agent used as food additive to prevent diarrhea in calve and swine (Magyar et al., J. Biol. Chem, 1999, 274, 7316-7324). Gliotoxin has immunosuppressive properties which were evaluated for the selective *ex vivo* removal of immune cells responsible for tissue rejection (Waring et al., Gen. Pharmacol., 1996, 27, 1311-1316). Several compounds such as ambewelamides, verticillin and phenylahistin exhibit antitumour activities involving various mechanisms (Chu et al., J. Antibiot. (Tokyo), 1995, 48, 1440-1445 ; Kanoh et al., J. Antibiot. (Tokyo), 1999, 52, 134-141; Williams et al., Tetrahedron Lett., 1998, 39, 9579-9582).

Many others like albonoursin produced by *Streptomyces noursei,* display antimicrobial activities (Fukushima et al., J. Antibiot. (Tokyo), 1973, 26, 175-176). Cyclo(Tyr-Tyr) and cyclo(Tyr-Phe) were shown to be potential cardioactive agents: cyclo(Tyr-Tyr) being a potential cardiac stimulant and cyclo(Tyr-Phe) being a cardiac inhibitor (Kilian et al., Pharmazie, 2005, 60, 305-309). These two cyclodipeptides were also tested as receptor interacting agents and the two compounds were found to exhibit significant binding to opioid receptors (Kilian et al., 2005, precited). Moreover, they were evaluated as antineoplastic agents and cyclo(Tyr-Phe) was shown to induce growth inhibition of three different cultured cell lines (Kilian et al., 2005, precited). It has been described that the cyclo(ΔAla-L-Val) produced by *Pseudomonas aeruginosa* could be involved in interbacterial communication signals (Holden et al., Mol. Microbiol., 1999, 33, 1254-1266). Other compounds are described as being involved in the virulence of pathogenic microorganisms or else as binding to iron or as having neurobiological properties (King et al., J. Agr. Food Chem., 1992, 40, 834-837; Sammes, Fortschritte der Chemie Organischer Naturstoffe, 1975, 32, 51-118; Alvarez et al., J. Antibiot., 1994, 47, 1195-1201).

Although the number of known DKPs is increasing steadily, biosynthesis pathways of these compounds are still largely unexplored, leading to little knowledge regarding their synthesis.

In several cases reported so far, the formation of DKPs occurs spontaneously from linear dipeptides for which the cis-conformation of the peptide bond is favoured by the presence of an N-alkylated amino acid or a proline residue. Such spontaneous cyclisation has also been observed in the course of non ribosomal peptide synthesis of gramicidin S and tyrocidine A in *Bacillus brevis,* due to the instability of the thioester linkage during peptide elongation on peptide synthetase megacomplexes (Schwarzer et al., Chem. Biol, 2001, 8, 997-1010). Thus, in all of the known mechanisms of spontaneous DKP formation, the primary structure of the precursor dipeptide, in particular the conformation of its peptide bond, appears to be a fundamental requirement for the formation of the DKP ring to take place and for the process to result in the production of the final DKP derivative.

However, such a spontaneous cyclisation reaction cannot account for the biosynthesis of the large majority of DKP derivatives that do not contain a proline residue or an N-alkylated residue.

Known methods for producing DKP-derivatives include chemical synthesis, extraction from natural producer organisms and also enzymatic methods:
- Chemical methods can be used for synthesizing DKP derivatives (Fischer, J. Pept. Sci., 2003, 9, 9-35) but they are considered to be disadvantageous in respect of cost and efficiency as they often necessitate the use of protected amino acyl precursors and lead to the loss of stereochemical integrity. Moreover, they are not environment-friendly methods as they use large amounts of organic solvents and the like.
- Extraction from natural producer organisms can be used but the productivity remains low because the contents of desired DKP-derivatives in natural products are often low.
- Enzymatic methods, *i.e*. methods utilizing enzymes either *in vivo* (*e.g*. culture of microorganisms expressing cyclodipeptide-synthesizing enzymes or microorganism cells isolated from the culture medium) or *in vitro* (*e.g.* purified cyclodipeptide-synthesizing enzymes) can be used. Enzymes known to produce cyclodipeptides are non-ribosomal peptide synthetases (hereinafter referred to as NRPS) (Gruenewald et al., Appl. Environ. Microbiol., 2004, 70, 3282-3291) and AlbC which is a cyclodipeptide synthetase (CDS) (Lautru et al., Chem. Biol., 2002, 9, 1355-1364; International Application WO 2004/000879):
   the enzymatic method utilizing NRPS has already been reported to produce a specific cyclodipeptide. The two genes coding .for the bimodular complex TycA/TycB1 from *Bacillus brevis* (Mootz and Marahiel, J. Bacteriol., 1997, 179, 6843-6850) were coexpressed in *Escherichia coli* and gave rise to the production of cyclo(DPhe-Pro) (Gruenewald et al., Appl. Environ. Microbiol., 2004, 70, 3282-3291). The cyclodipeptide was stable, not toxic to *E. coli* and secreted in the culture medium. However, the methods utilizing NRPS appear essentially restricted to the production of cyclodipeptides containing N-alkylated residues. Indeed, the formation of DKP derivatives occurs spontaneously from linear dipeptides for which the *cis* conformation of the peptide bond is favored by the presence of prolyl residues (Walzel et al., Chem. Biol., 1997, 4, 223-230 ; Schwarzer et al., Chem. Biol., 2001, 8, 997-1010) or N-methylated residues (Healy et al., Mol. Microbiol., 2000, 38, 794-804). Moreover, the methods utilizing NRPS are difficult to implement: NRPS being large multimodular enzyme complexes, they are not easy to manipulate both at the genetic or biochemical levels.
   the enzymatic method utilizing AlbC was also described to produce specific cyclodipeptides. The expression of Albs from *Streptomyces noursei* by heterologous hosts *Streptomyces lividans* TK21 or *E. coli* led to the production of two cyclodipeptides, cyclo(Phe-Leu) and cyclo(Phe-Phe) that were secreted in the culture medium (Lautru et al., Chem. Biol., 2002, 9, 1355-1364). AlbC catalyzes the condensation of two amino acyl derivatives to form cyclodipeptides, containing or not containing N-alkylated residues, by an unknown mechanism. This unambiguously shows that a specific enzyme unrelated to non ribosomal peptide synthetases can catalyze the formation of DKP derivatives: AlbC is the first example of an enzyme that is directly involved in the formation of the DKP motif.

Furthermore the obtained cyclo(Phe-Leu) cyclodipeptide may be transformed into a cyclo(α,β-dehydro-dipeptide), i.e. albonoursin, or cyclo(ΔPhe-ΔLeu), an antibiotic produced by *Streptomyces noursei,* in the presence of cyclic dipeptide oxydase (CDO) which specifically catalyzed the formation of albonoursin, in a two-step sequential reaction starting from the natural substrate cyclo(L-Phe-L-Leu) leading first to cyclo(ΔPhe-L-Leu) and finally to cyclo(ΔPhe-ΔLeu) corresponding to albonoursin (Gondry et al., Eur. J. Biochem., 2001, 268, 1712-1721). Said CDO may also transform various cyclodipeptides into α,β-dehydrodipeptides (Gondry et al., Eur. J. Biochem., 2001, precited).

The DKP derivatives exhibit various biological functions, making them useful entities for the discovery and development of new drugs, food additives and the like. Accordingly, it is necessary to be able to have large amounts of these compounds available.

An understanding of the pathways for the natural synthesis of the diketopiperazine derivatives could enable a reasoned genetic improvement in the producer organisms, and would open up perspectives for substituting or improving the existing processes for synthesis (via chemical or biotechnological pathways) through the optimization of production and purification yields. In addition, modification of the nature and/or of the specificity of the enzymes involved in the biosynthetic pathway for the diketopiperazine derivatives could result in the creation of novel derivatives with original molecular structures and with optimized biological properties.

The Inventors have now identified new cyclodipeptide synthesizing enzymes (or cyclodipeptide synthetases or CDS), said enzymes sharing at least 34% of identity or at least 56% of similarity with at least one of the polypeptide of the sequences SEQ ID N°: 2, SEQ ID N°: 4, SEQ ID N°: 6 and SEQ ID N°: 8, and said enzymes being able to catalyse the specific formation of cyclo(Leu-Leu) cyclodipeptide.

These percentages of sequence identity and sequence similarity defined herein were obtained using the BLAST program (blast2seq, default parameters) (Tatutsova and Madden, FEMS Microbiol Lett., 1999, 174, 247-250).

Said percentages derived from the comparison of the full-length sequences SEQ ID N°: 2, SEQ ID N°: 4, SEQ ID N°: 6 and SEQ ID N°: 8 one with another, as described in Table V; preferably said percentages derived by calculating them on an overlap representing a percentage of length of said sequences as specified in Table V.

The polypeptide of SEQ ID N°: 2 corresponds to the polypeptide YvmC_{sub} of *Bacillus subtilis* subsp. *subtilis* strain 168 (GenBank accession number Acc. n° CAB15512). The polypeptide of SEQ ID N° 4 corresponds to the polypeptide Plu0297 of *Photorhabdus luminescens* subsp. *laumondii* TTO1 (Genbank Acc. n° Q7N9M5). The polypeptide of sequence SEQ ID N° 6 corresponds to the polypeptide YvmCₜₕᵤ of *Bacillus thuringiensis* serovar *israelensis* strain 1884, and shares 94% of identity and 98% of similarity with the polypeptide RBTH_07362 of *Bacillus thuringiensis* serovar *israelensis* ATCC 35646 (Genbank Acc. n° EAO57133). The polypeptide of SEQ ID N° 8 corresponds to the polypeptide YvmC_{lic} of *Bacillus licheniformis* ATCC 14580 (Genbank Acc. n° AAU25020). The informations available on the different databases concern hypothetical proteins which were not isolated and for which no function has been defined.

Thus one object of the invention is the use of an enzyme selected from the group consisting of the polypeptides of sequences SEQ ID N°2, SEQ ID N°4, SEQ ID N°6 and SEQ ID N°8, for the synthesis of a cyclo(Leu-Leu) cyclodipeptide.

Another object of the invention is the use of a polynucleotide selected from:
a) a polynucleotide encoding a cyclodipeptide synthetase as defined above;
b) a complementary polynucleotide of the polynucleotide a);
c) a polynucleotide which hybridizes to polynucleotide a) or b) under stringent conditions, and
d) a polynucleotide encoding a cyclodipeptide synthethase selected from the group consisting of the polynucleotides of sequences SEQ ID N°1, SEQ ID N°3, SEQ ID N°5 and SEQ ID N°7,
   for the synthesis of a cyclo(Leu-Leu) cyclodipeptide.

The polynucleotides of sequences SEQ ID N° 1, SEQ ID N°3, SEQ ID N°5 and SEQ ID N°7 encode respectively the polypeptides of sequences SEQ ID N° 2, SEQ ID N° 4, SEQ ID N° 6 and SEQ ID N° 8.

The term "hybridize(s)" as used herein refers to a process in which polynucleotides hybridize to the recited nucleic acid sequence or parts thereof. Therefore, said nucleic acid sequence may be useful as probes in Northern or Southern Blot analysis of RNA or DNA preparations, respectively, or can be used as oligonucleotide primers in PCR analysis dependent on their respective size. Preferably, said hybridizing polynucleotides comprise at least 10, more preferably at least 15 nucleotides while a hybridizing polynucleotide of the present to be used as a probe preferably comprises at least 100, more preferably at least 200, or most preferably at least 500 nucleotides.

It is well known in the art how to perform hybridization experiments with nucleic acid molecules, i.e. the person skilled in the art knows what hybridization conditions she/he has to use in accordance with the present invention. Such hybridization conditions are referred to in standard text books such as Sambrook et al., Molecular Cloning: A Laboratory Manual; Cold Spring Harbor Laboratory Press, 2nd edition 1989 and 3rd edition 2001; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002 and other standard laboratory manuals known by the person skilled in the Art or as recited above. Preferred in accordance with the present inventions are stringent hybridization conditions.

"Stringent hybridization conditions" refer, e.g. to an overnight incubation at 42°C in a solution comprising 50% formamide, 5×SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed e.g. by washing the filters in 0.2 x SSC at about 65°C.

Also contemplated are nucleic acid molecules that hybridize at low stringency hybridization conditions. Changes in the stringency of hybridization and signal detection are primarily accomplished through the manipulation of formamide concentration; salt conditions, or temperature. For example, lower stringency conditions include an overnight incubation at 37°C in a solution comprising 6 x SSPE (20 x SSPE = 3 mol/l NaCl; 0.2 mol/l NaH₂PO₄; 0.02 mol/l EDTA, pH 7.4), 0.5% SDS, 30% formamide, 100 µg/ml salmon sperm blocking DNA; followed by washes at 50°C with 1 x SSPE, 0.1% SDS.

In addition, to achieve even lower stringency, washes performed following stringent hybridization can be done at higher salt concentrations (e.g. 5 x SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations.

Another object of the invention is an isolated polynucleotide selected from:
a) a polynucleotide encoding a cyclodipeptide synthetase as defined above;
b) a complementary polynucleotide of the polynucleotide a);
c) a polynucleotide which hybridizes to polynucleotide a) or b) under stringent conditions.

Advantageously, said isolated polynucleotide is selected from the group consisting of the polynucleotides of sequences SEQ ID N°1, SEQ ID N° 3, SEQ ID N°5 and SEQ ID N° 7.

Said polynucleotides can be obtained from DNA libraries, particularly from microorganism DNA libraries. For example the polynucleotide having the sequence SEQ ID N° 1 can be obtained from a *Bacillus subtilis* DNA library, the polynucleotide having the sequence SEQ ID N° 3 can be obtained from a *Photorhabdus luminescens* DNA library, the polynucleotide having the sequence SEQ ID N° 5 can be obtained from a *Bacillus thuringiensis* DNA library, the polynucleotide having the sequence SEQ ID N° 7 can be obtained from a *Bacillus licheniformis* DNA library. Said polynucleotides can also be obtained by means of a polymerase chain reaction (PCR) carried out on the total DNA of the respective microorganisms, or can be obtained by RT-PCR carried out on the total RNA of the same microorganism.

Another object of the invention is the use of a recombinant vector characterized in that comprises a polynucleotide as defined above, for the synthesis of a cyclo(Leu-Leu) cyclodipeptide.

Said vector may be any known vector of the prior art, and is preferably an expression vector. As vectors that can be used according to the invention, mention may in particular be made of plasmids, cosmids, bacterial artificial chromosomes (BACs), integrative elements of actinobacteria, viruses or bacteriophages.

Said vector may also comprise any regulatory sequences required for the replication of the vector and/or the expression of the polypeptide encoded by the polynucleotide (promoter, termination sites, etc...).

Another object of the invention is the use of a modified host cell comprising a polynucleotide as defined above, for the synthesis of a cyclo(Leu-Leu) cyclodipeptide.

Such a modified host cell may be any known heterologous expression system using prokaryotes or eukaryotes as hosts, and is preferably a prokaryotic cell. By way of example, mention may be made of animal or insect cells, and preferably of a microorganism and in particular a bacterium such as *Escherichia coli.*

The introduction of the polynucleotide or the recombinant vector according to the invention into the host cell to be modified can be carried out by any known method, such as, for example, transfection, infection, fusion, electroporation, microinjection or else biolistics.

In another aspect, the present invention relates to a method for the synthesis of cyclo(Leu-Leu) cyclodipeptide characterized in that it comprises the steps of:
(1) incubating leucine, under suitable conditions, with at least one cyclodipeptide synthetase enzyme as defined above, and
(2) recovering the cyclo(Leu-Leu) cyclodipeptide thus obtained.

The term "suitable conditions" is preferably intended to mean the appropriate conditions (concentrations, pH, buffer, temperature, time of reaction, etc...) under which the amino acids and the cyclodipeptide synthetase are incubated, to allow the synthesis of cyclodipeptides in an appropriate buffer.

An example of an appropriate concentration of amino acids and cyclodipeptide synthetase is the following: leucine at a concentration of between 0.1 mM and 100 mM, preferably of between 1 mM and 10 mM; the cyclodipeptide synthetase as defined above (*e.g*. polypeptide of a sequence selected from SEQ ID N° 2, SEQ ID N° 4, SEQ ID N° 6 and SEQ ID N° 8) is at a concentration of between 0.1 nM and 100 µM, preferably of between 1 µM and 100 µM.

An example of an appropriate buffer is 100 mM Tris-HCl, containing 150 mM NaCl, 10 mM ATP, 20 mM MgCl₂, supplemented with a soluble prokaryote cell extract.

Appropriate pH is ranging between 6 and 8, appropriate temperature is ranging between 20 and 40°C, and appropriate reaction time is ranging between 12 and 24 hours.

Another object of the present invention is the method for the synthesis of α,β-dehydrogenated cyclo(Leu-Leu) cyclodipeptide, characterized in that it comprises:
(1') incubating leucine, under suitable conditions, with a cyclodipeptide synthetase enzyme as defined above and a purified CDO, and
(2') recovering the α,β-dehydrogenated cyclopeptides.

Therefore, according to a prefered embodiment of carrying out said method, step (1) or step (1') is performed in presence of Leu at a concentration between 0.1 mM and 100 mM, preferably of 1 mM to 10 mM, said cyclodipeptide synthetase as defined hereabove at a concentration between 0.1 nM and 100 µM, preferably of 1 µM to 100 µM, in a buffer at pH ofbetween 6 and 8, and containing a soluble extract of prokaryote cells such as *E. coli* or *Streptomyces* cells which does not produce cyclodipeptide synthetase.

α,β-dehydrogenated cyclo(Leu-Leu) cyclopeptide derivative may be obtained from the hereabove described cyclodipeptide, according to the method described in Gondry et al. (Eur. J Biochem., 2001, precited) or in the International PCT Application WO 2004/000879.

For example, an amount of 5 10⁻³ units of CDO is added to the buffer used according to the method described above. One unit of CDO was defined as the amount catalyzing the formation of 1 µmol of cyclo(ΔPhe-His) per min under standard assay conditions (Gondry et al., Eur. J. Biochem., 2001, 268, 4918-4927).

According to another preferred embodiment of the method for the synthesis of cyclo(Leu-Leu) cyclodipeptide or α,β-dehydrogenated derivative thereof, it further comprises prior or simultaneously with step (1) or step (1'), a step consisting in the use of a polynucleotide as defined hereabove, for synthesizing said cyclodipeptide synthetase.

The methods of synthesis of cyclo(Leu-Leu) cyclodipeptide or α,Δ-dehydrogenated derivative thereof may be carried out in any suitable biological system, particularly in a host such as, for example, a microorganism, for instance a bacterium such as *Escherichia coli* or *Streptomyces lividans,* or any known heterologous expression system using prokaryotes or eukaryotes as hosts, or even *in vitro* acellular systems.

Another object of the invention is a method for producing cyclo(Leu-Leu) cyclodipeptide, comprising the following steps:
(1') culturing a host cell, as defined above in suitable culture conditions for said host cell, and
(2') recovering the cyclo(Leu-Leu) cyclodipeptide from the culture medium.
   α,β-dehydrogenated cyclo(Leu-Leu) cyclodipeptide derivative may be obtained in the following conditions:
(1') culturing a modified host cell as defined here above in appropriate culture conditions for said host cell,
(1") incubating the cyclo(Leu-Leu) cyclodipeptide obtained from step (1') with a purified CDO, and
(2") recovering the α,β-dehydrogenated cyclo(Leu-Leu) cyclodipeptide derivative from the culture medium.

The conditions for using CDO are the same than those described above.

The recovering of the cyclo(Leu-Leu) cyclodipeptide or of the α,β-dehydrogenated derivative thereof can be carried out directly from synthesis by means of liquid-phase extraction techniques or by means of precipitation, or thin-layer or liquid-phase chromatography techniques, in particular reverse-phase HPLC, or any method suitable for purifying peptides, one known to those skilled in the art.

Besides the above provisions, the invention also comprises other provisions which will emerge from the following description, which refers to examples of implementation of the invention and also to the attached drawings, in which:
- Figure 1(a) represents the structure of piperazine-2,5-dione cycle. The cis-amide bond is in bold. Figure 1(b) represents the structure of cyclo(Leu-Leu).
- Figure 2 illustrates the cloning strategy for the construction of the expression vector pEXP-YvmC_{sub.}
- Figure 3 represents the HPLC analysis of the culture media of *E. coli* BL21 AI cells expressing the YvmC_{sub}, YvmCmₜₕᵤ or YvmC_{lic} proteins. Culture supernatants of cells transformed with:
   (————) ) pEXP-YvmC_{sub},,
   (— · — ) pEXP-YvmC_{thu,}
   (..........) pEXP-YvmC_{lic}, and as a control,
   (————) ) empty pQE60,
   were analyzed by RP-HPLC. The chromatograms were recorded at 220 nm.
- Figure 4 illustrates the HPLC analysis of the culture media of *E. coli* BL21 AI cells expressing the Plu0297 protein. Culture supernatants of cells transformed with:
   (————) pEXP-Plu0297, and as a control
   (————) empty pQE60,
   were analyzed by RP-HPLC. The chromatograms were recorded at 220 nm.
- Figure 5 illustrates the MS (a) and MSMS (b) spectra of collected fraction of peak B_{sub}. Collected fraction was directly infused into the mass spectrometer and full scan MS acquired on line (Figure 5a). Main m/z peak at 227.0± 0.1 was isolated as parent ion and subjected to MSMS fragmentation giving rise to a daughter ion spectrum (Figure 5b). Encircled peak m/z at 86.3 matches to immonium ion of leucine or isoleucine, respectively referred to as iLeu and iIle.
- Figure 6 illustrates the MS spectrum (a) and MSn spectra (b) of collected fraction of peak Bₜₕᵤ. Collected fraction was directly infused into the mass spectrometer and full scan MS acquired on line (Figure 6a). Main m/z peak at 227.0± 0.1 was isolated as parent ion and subjected to MSMS fragmentation giving rise to a daughter ion spectrum (Figure 6b). Encircled peak m/z at 86.3 matches to immonium ion of leucine or isoleucine, respectively referred to as iLeu and iIle.
- Figure 7 illustrates the MS spectrum (a) and MSn spectra (b) of collected fraction of peak B_{lic}. Collected fraction was directly infused into the mass spectrometer and full scan MS acquired on line (Figure 7a). Main m/z peak at 227.0± 0.1 was isolated as parent ion and subjected to MSMS fragmentation giving rise to a daughter ion spectrum (Figure 7b). Encircled peak m/z at 86.3 matches to immonium ion of leucine or isoleucine, respectively referred to as iLeu and iIle.
- Figure 8 illustrates the MS spectrum (a) and MSn spectra (b) of collected fraction of peak D. Collected fraction was directly infused into the mass spectrometer and full scan MS acquired on line (Figure 8a). Main m/z peak at 227.0± 0.1 was isolated as parent ion and subjected to MSMS fragmentation giving rise to a daughter ion spectrum (Figure 8b). Encircled peak m/z at 86.3 matches to immonium ion of leucine or isoleucine, respectively referred to as iLeu and iIle.
- Figure 9 illustrates the identification of the cyclodipeptides produced by YvmCsub-like and Plu0297 enzymes. *E. coli* BL21 AI cells expressing:
   (———) YvmC_{sub} from *B. subtilis,* and
   (............) Plu0297 from *P. luminescens,*
   were grown in M9 medium.

The two culture supernatants were then analyzed by RP-HPLC. The cyclodipeptide standards:
(———) ) cyclo(Ile-Ile),
(— · —) cyclo(Ile-Leu), and
(............) cyclo(Leu-Leu),
   were also analyzed. The chromatograms were recorded at 220 nm (left scale for standards and right scale for assays).

It should be clearly understood, however, that these examples are given only by way of illustration of the subject of the invention, of which they in no way constitute a limitation.

### EXAMPLE 1: CONSTRUCTION OF AN ESCHERICHIA COLI EXPRESSION VECTOR ENCODING THE YvmC_{sub} PROTEIN

As several homologues of YvmC from *Bacilli* are studied, these homologues were called YvmC_{sub} for the one from *Bacillus subtilis* sub. *subtilis* strain 168, YvmC_{lic} for the one from *Bacillus licheniformis* ATCC 14580 and YvmCₜₕᵤ for the one from from *Bacillus thuringiensis* serovar *israelensis* **1884**.

The expression vector encoding the said protein mentioned above were constructed using the Gateway™ cloning technology (Invitrogen). They were designed to express each protein as a cytoplasmic fusion protein carrying at its N-terminus end a (His)₆ tag, the translated sequence of the *attB* recombination site (necessary for cloning) and the TEV protease cleavage site.

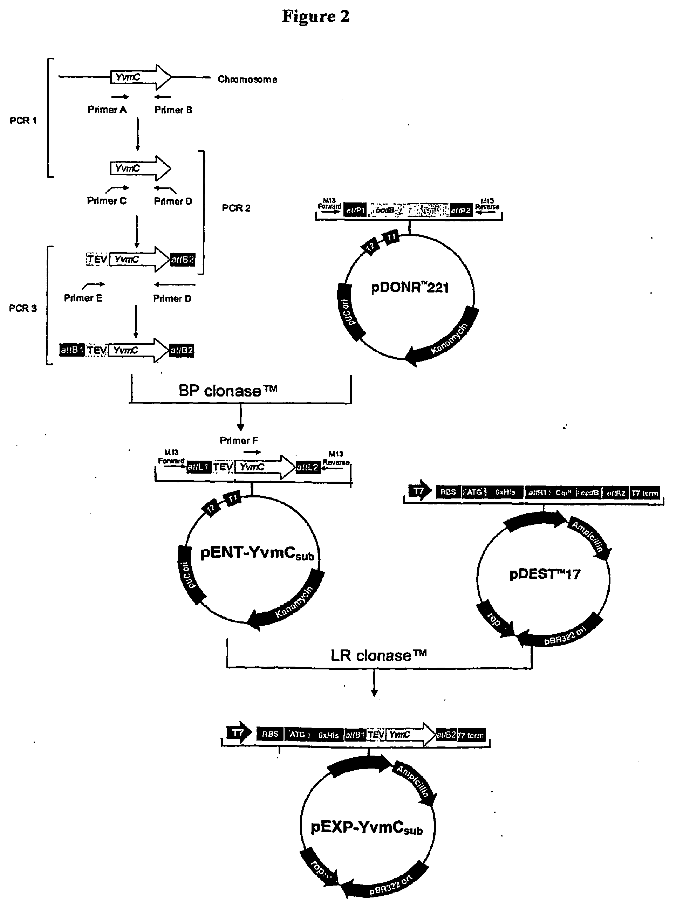

The whole cloning strategy used to construct the expression vector encoding the YvmC_{sub} protein (Acc. n° CAB15512) is shown in Figure 2. First, the *attB*-flanked DNA suitable for recombinational cloning and encoding the YvmC_{sub} protein was obtained after three successive PCR. The *YvmC_{sub}* gene was amplified in the first PCR (PCR 1 in Figure 2) using primers A and B as described in Table I and, as template, genomic DNA of *Bacillus subtilis subsp. subtilis* strain 168 whose genome has been completely sequenced (Acc. n° AL009126). The PCR conditions were one initial denaturation step at 97°C for 4 minutes followed by 25 cycles at 94°C for one minute, 54°C for one minute, 72°C for 2 minutes, and one final extension step at 72°C for 10 minutes. The reaction mixture (50 µl) comprised 1 µl of chromosomal DNA (25 ng/µl), 0.3 µl of each primer solution at 100 µM, 5 µl of 10X *Pfu* DNA polymerase buffer with MgSO₄ (provided by the *Pfu* DNA polymerase supplier), 0.1 µl of a mix of dNTPs 10 mM each and 1 µl of *Pfu* DNA polymerase (2.5 U/µl; Fermentas). The PCR product (herein after referred to as *"PCR product 1"*) was then purified using the "GFX PCR DNA and Gel Band Purification" kit (Amersham Biosciences) after electrophoresis in 1% agarose gel (16). The second PCR (PCR 2 in Figure 2) enabled the addition of the sequence encoding the TEV protease cleavage site to the *PCR product 1* 5' terminus and that of the *attB2* encoding sequence to the 3' terminus. PCR conditions were one initial denaturation step at 95°C for 5 minutes followed by 30 cycles at 95°C for 45 seconds, 50°C for 45 seconds, 72°C for 1.5 minutes, and one final extension step at 72°C for 10 minutes. The reaction mixture (50 µl) comprised 5 ng *PCR product 1,* 0.4 µM of primers C and D as described in Table 1, 2.5 units Expand High Fidelity Enzyme mix (Roche), 1X Expand High Fidelity buffer with 1.5 mM MgCl₂ (Roche) and 200 µM each dNTP. After electrophoresis in 1% agarose gel and purification with the QIAquick Gel Extraction kit (Qiagen), the PCR product (hereinafter referred to as *"PCR product* 2") was used for the third PCR (PCR 3 in Figure 2) that enabled the addition to the *PCR product 2* 5' terminus of the *attB1* encoding sequence. PCR was carried out as described above using 5 ng *PCR product 2* as a template and 0.4 µM of primers E and D as described in Table I. The resulting PCR product (hereinafter referred to as *"PCR product* 3") was purified as previously described.

Second, the *attB*-flanked *PCR product 3* was recombined with the pDONR™221 donor vector (Invitrogen) in BP clonase™ reaction to generate the entry vector pENT-YvmC_{sub}. pENT-YvmC_{sub} was sequenced between the two-recombination sites using ABI PRISM 310 Genetic Analyzer (Applied Biosystem) and primers M13 forward, M13 reverse and F as described in Table 1. pENT-YvmC_{sub} and the commercial destination vector pDEST-17 (Invitrogen) were used in LR clonase™ subcloning reaction to generate the expression vector pEXP-YvmC_{sub} (SEQ ID N°26) following the supplier recommendations. The recombination mixture was used for transformation of *E. coli* DH5α chemically competent cells and a positive clone was selected after analysis by colony PCR. The 50 µl reaction mix comprised a small amount of colony as a template, 200 µM each dNTP, 0.2 µM primer M13 forward and M13 reverse, 1X ThermoPol Reaction Buffer (New England Biolabs) and 1.25 unit *Taq* DNA Polymerase (New England Biolabs). The PCR conditions used were the following: one initial denaturation step at 95°C for 5 minutes followed by 30 cycles at 92°C for 30 seconds, 50°C for 30 seconds, 72°C for 2 minutes. Plasmid DNA was isolated from positive clones using the Wizard DNA Purification System (Promega) and conserved at -20°C.

**Table I. Primers used to construct the expression vector pEXP-YvmC_{sub}.**

| **Name** | **Corresponding sequence (5' to 3')** |
|---|---|
| A | AAGTTTTAGGGGTGAATGAGATGACCGGAATGG (SEQ ID N°9) |
| B | CGAAGCTTACTCCCCCTATCATCCTTCAGATGTGA (SEQ ID N° 10) |
| C | GGCTTCGAGAATCTTTATTTTCAGGGCACCGGAATGGTAACG (SEQ ID N° 11) |
| D | GGGGACCACTTTGTACAAGAAAGCTGGGTCCTTATCCTTCAGATGTGATCCG (SEQ ID NO: 12) |
| E | GGGGACAAGTTTGTACAAAAAAGCAGGCTTCGAGAATCTTTATTTTC (SEQ ID N° 13) |
| F | ATGAGGCGGCTAATC-17CTA (SEQ ID N° 14) |
| M13 Forward | GTAAACGACGGCCAG (SEQ ID N°15) |
| M13 Reverse | CAGGAAACAGCTATGAC (SEQ ID N°16) |

### EXAMPLE 2: CONSTRUCTION OF AN ESCHERICHIA COLI EXPRESSION VECTOR ENCODING THE Plu0297 PROTEIN

The same cloning strategy was applied to construct the expression vector encoding Plu0297 (Acc. n° Q7N9M5). As template genomic DNA isolated from *Photorhabdus luminescens subsp. laumondii* TTO1 was used, whose genome has been sequenced (Acc. n° BX470251) and the primers A, B, C, D, and E quoted in Table II to generate PCR product suitable for recombinational cloning. Then, pENT-Plu0297 and pEXP-Plu0297 were obtained after recombination with BP clonase™ and LR clonase™ as described above. DNA sequence of pENT-Plu0297 was verified by using the primers M13 forward and M13 reverse quoted in Table I and primer F described in Table II. Plasmid pEXP-Plu0297 (sequence SEQ ID N°27) was isolated from a positive clone obtained after colony PCR analysis as previously described.

**Table II. Primers used to construct the expression vector pEXP-Plu0297.**

| **Name** | **Corresponding sequence (5' to 3')** |
|---|---|
| A | AACGAACAATCAAATATTATCAGCCCATTCAACATTGCTG (SEQ ID N°17) |
| B | CGTATTAACTTTAAACGCAGTGACTATTTCATCAGACTGT(SEQ ID N°18) |
| C | GGCTTCGAGAATCTTTATTTTCAGGGCCTGCACGAGAATTCACC (SEQ ID N°19) |
| D | GGGGACCACTTTGTACAAGAAAGCTGGGTCCTTATAATTGAGTGACGATTCCG (SEQ ID N°20) |
| E | GGGGACAAGTTTGTACAAAAAAGCAGGCTTCGAGAATCTTTATTTTC (SEQ ID N°13) |
| F | GCCGGTGATAAAGTAACGAT (SEQ ID N°21) |

### EXAMPLE 3: CONSTRUCTION OF AN ESCHERICHIA COLI EXPRESSION VECTOR ENCODING THE YvmCₜₕᵤ AND YvmC_{lic} PROTEINS

For creating the expression vector encoding YvmCₜₕᵤ from *Bacillus thuringiensis* serovar *israelensis* **1884** and YvmC_{lic} (Acc. n° AAU25020) from *Bacillus licheniformis* ATCC 14580, a slightly different strategy from the one used for YvmC_{sub} was used: the first PCR step (PCR1 in Figure 2) was omitted, and DNA encoding YvmCₜₕᵤ or YvmC_{lic} was directly amplified from corresponding chromosomal DNA by using oligonucleotides C and D described in Tables III and IV, respectively. Chromosomal DNA isolated from *Bacillus thuringiensis israelensis 1884* for YvmCₜₕᵤ and chromosomal DNA isolated from *Bacillus licheniformis* ATCC 14580 were used, whose genome has been completely sequenced (Acc. n° CP000002), for YvmC_{lic}. Constructions and verifications of the corresponding pENT and pEXP vectors (sequence SEQ ID N°28 for pEXP-YvmCₜₕᵤ and sequence SEQ ID N°29 for pEXP-YvmC_{lic}) were made as described for YvmC_{sub} and Plu0297.

**Table III. Primers used to construct the expression vector pEXP-YvmCₜₕᵤ.**

| **Name** | **Corresponding sequence (5' to 3')** |
|---|---|
| C | GGCTTCGAGAATCTTTATTTTCAGGGCACGAATGCTATAGCGGTAAG (SEQ ID N°22) |
| D | GGGGGGGGGACCACTTTGTACAAGAAAGCTGGGTCCTTAAGATAAATTTTCCATTTCTTGTACCA (SEQ ID N°23) |
| E | GGGGACAAGTTTGTACAAAAAAGCAGGCTTCGAGAATCTTTATTTTC(SEQ ID N°13) |

**Table IV. Primers used to construct the expression vector pEXP-YvmC_{lic}.**

| **Name** | **Corresponding sequence (5' to 3')** |
|---|---|
| C | GGCTTCGAGAATCTTTATTTTCAGGGCACAGAGCTTATAATGG (SEQ ID N°24) |
| D | GGGGGGGGGACCACTTTGTACAAGAAAGCTGGGTCCTTATACTCGTTCCTCCTGCATGC (SEQ ID N°25) |
| E | GGGGACAAGTTTGTACAAAAAAGCAGGCTTCGAGAATCTTTATTTTC (SEQ ID N°13) |

### EXAMPLE 4: BACTERIAL CULTURES FOR PRODUCTION OF CYCLODIPEPTIDES

The same procedure was applied for expressing all the four proteins, YvmC_{sub}, YvinCₜₕᵤ, YvmC_{lic} and Plu0297. Bacteria were grown in minimal M9 medium (6 g/l Na₂HPO₄, 3 g/l KH₂PO₄, 0.5 g/l NaCl, 1 g/l NH₄Cl, 1mM MgSO₄, 0.1 mM CaCl₂, 1 µg/ml thiamine and 0.5% glucose or glycerol) (Sambrook et al., (2001) Molecular Cloning: A Laboratory Manual, New York) supplemented with 1 ml of a vitamins solution and 2 ml of an oligoelements solution per liter of minimal medium. Vitamins solution contains 1.1 mg/l biotin, 1.1 mg/l folic acid, 110 mg/l para-aminobenzoic acid, 110 mg/l riboflavin, 220 mg/l pantothenic acid, 220 mg/l pyridoxine-HCl, 220 mg/l thiamine and 220 mg/l niacinamide in 50% ethanol. Oligoelements solution was made by diluting a FeCl₂-containing solution 50 fold in H₂O. The FeCl₂-containing solution contains for 100 ml: 8 ml concentrated HCl, 5g FeCl₂.4H₂O, 184 mg CaCl₂.2H2O, 64 mg H₃BO₃, 40 mg MnCl₂.4H₂O, 18 mg CoCl₂.6H₂O, 4 mg CuCl₂.2H₂O, 340 mg ZnCl₂, 605 mg Na₂MoO₄.2H₂O.

The whole process was performed using standard large-scale procedures and materials (*e.g*. Erlen Meyer flasks). 50 µl chemically competent BL21AI cells (Invitrogen) were transformed with 20 ng plasmid using standard heat-shock procedure (Sambrook et al., aforementioned). After 1 h outgrowth in SOC medium (Sambrook et al., aforementioned) at 37°C, bacteria were spread on LB plates containing 200 µg/ml ampicillin. A few colonies were picked up to inoculate M9 liquid medium supplemented with vitamins and oligoelements solutions containing 0.5% glucose and 200 µg/ml ampicillin. After overnight incubation at 37°C with shaking, 500 µl of the starter cultures were used to inoculate 25 ml M9 minimal medium supplemented with vitamins and oligoelements solutions containing 0.5% glycerol and 200 µg/ml ampicillin. Cultures were grown at 37°C until OD₆₀₀ ∼ 0.5 and 0.02% L-arabinose was added. Cultures were continued at 20°C for 24 h. Cultures supernatants were collected after centrifugation at 2,500 g for 20 min and kept at -20°C.

As a control experiment, we used BL21AI cells transformed by pQE60 (Qiagen), an ampicillin resistance gene-carrying vector that expresses no cyclodipeptide synthesizing enzyme. Growth and supernatant collection were conducted as described above.

### EXAMPLE 5: COMPARISON OF PEPTIDE SEQUENCES

The sequences of the proteins YvmC_{sub}, YvmC_{lic}, YvmCₜₕᵤ and Plu0297 were compared. Two programs were used to measure the similarity/identity of those proteins one with another. The results are presented in Table V.

**Table V: Comparison of sequences**

| | YvmC_{sub} (248 aa) | YvmC_{lic} (249 aa) | YvmCₜₕᵤ (239 aa) | Plu0297 (234 aa) |
|---|---|---|---|---|
| YvmC_{sub} 248 aa | 100/100 | Identities = 174/247 (70%), Positives = 201/247 (81%), Gaps = 0/247 (0%) | Identities = 147/238 (61%), Positives = 183/238 (76%), Gaps = 0/238 (0%) | Identities = 86/229 (37%), Positives = 129/229 (56%), Gaps = 3/229 (1%) |
| | | 70.445% of identity (86.640% similar) in 247 aa overlap | 61.765% of identity (84.874% similar) in 238 aa overlap | 37.555% of identity (68.996% similar) in 229 aa overlap |
| YvmC_{lic} 249 aa | | 100/100 | Identities = 155/236 (65%), Positives = 186/236 (78%), Gaps = 0/236 (0%) | Identities = 79/225 (35%), Positives = 133/225 (59%), Gaps = 3/225 (1%) |
| | | | 65.678% of identity (87.288% similar) in 236 aa overlap | 35.111 % of identity (70.667% similar) in 225 aa overlap |
| Yvmcₜₕᵤ 239 aa | | | 100/100 | Identities = 79/230 (34%), Positives = 132/230 (57%), Gaps = 3/230 (1%) |
| | | | | 34.348% of identity (70.435% similar) in 230 aa overlap |
| Plu0297 234 aa | | | | 100/100 |

| | | | | |
|---|---|---|---|---|
| **Bold characters**: result of comparisons with Blast (blast2seq, default parameters) / reference Tatusova and Madden, FEMS Microbiol. Lett., 1999, 174, 247-250). Normal size characters: result of comparisons with SSEARCH (default parameters) (matrix BLOSUM50)/ reference : Smith and Waterman, J. Mol. Biol., 1981, 147, 195-197; Pearson, Genomics, 1991, 11, 635-650). | | | | |

### EXAMPLE 6: DETECTION AND PURIFICATION OF A CYCLODIPEPTIDE IN THE CULTURE MEDIA BY HPLC ANALYSIS

Culture supernatants (200 µl) were acidified down to pH=3 with concentrated trifluoroacetic acid and then analyzed by HPLC. Samples were loaded onto a C18 column (4.6 x 250 mm, Vydac) and eluted with a linear gradient from 0% to 55% acetonitrile/deionized water with 0.1% trifluoroacetic acid for 60 min (flowrate, 1 ml/min). The elution was monitored between 220 and 500 nm using a diode array detector.

### EXAMPLE 7: IDENTIFICATION OF CYCLODIPEPTIDE DERIVATIVES BY MS AND MSMS ANALYSIS

HPLC-eluted fractions from culture supernatants (see above) were collected and analyzed by mass spectrometry using an ion trap mass analyzer Esquire HCT equipped with an orthogonal Atmospheric Pressure Interface-ElectroSpray Ionization (AP-ESI) source (Bruker Daltonik GmbH, Germany). The samples were directly infused into the mass spectrometer at a flow rate of 3 µl/min by means of a syringe pump. Nitrogen served as the drying and nebulizing gas while helium gas was introduced into the ion trap for efficient trapping and cooling of the ions generated by the ESI as well as for fragmentation processes. Ionization was carried out in positive mode with a nebulizing gas set at 9 psi, a drying gas set at 5 µl/min and a drying temperature set at 300°C. Ionization and mass analyses conditions (capillary high voltage, skimmer and capillary exit voltages and ions transfer parameters) were set for an optimal detection of compounds in the range of cyclodipeptides masses between 100 and 400 m/z. For MSMS experiments an isolation width of 1 mass unit was used for selecting the parent ion. Fragmentation amplitude was tuned until at least 90% of the isolated precursor ion was fragmented. Full scan MS and MSMS spectra were acquired using EsquireControl software and all data were processed using DataAnalysis software.

Different sources of cyclodipeptides were used as standards for mass and HPLC analyses. Cyclodipeptides cyclo(Leu-Leu) and cyclo(Phe-Leu) were respectively obtained from Sigma and Bachem. Cyclo(Ile-Leu) and cyclo(Ile-Ile) were chemically synthesised as described in Jeedigunta et al. (Tetrahedron, 2000, 56, 3303-3307).

### EXAMPLE 8: EXPRESSION OF YvmC_{sub}, YvmCₜₕᵤ, YvmC_{lic} OR Plu0297 IN E. COLI CYTOPLASM LEADS TO THE SYNTHESIS OF CYCLODIPEPTIDES

The YvmC_{sub}-encoding gene of *B. subtilis,* the YvmCₜₕᵤ-encoding gene of *B. thuringiensis israelensis 1884,* the YvmC_{lic}-encoding gene of *B. licheniformis* and the Plu0297-encoding gene of *P. luminescens* were respectively cloned in expression vectors, their expression in *E. coli* cytoplasm was induced and the cyclodipeptide synthesis activity *in vivo* was searched. First, in each case, the synthesis of a protein whose N-terminal sequence corresponded to the one expected for the corresponding (His)₆-tagged protein was observed. Then, the hypothetical formation of cyclodipeptides was investigated by analyzing the culture supernatants of *E. coli* cells expressing YvmC_{sub}, YvmCₜₕᵤ, YvmC_{lic} or Plu0297.

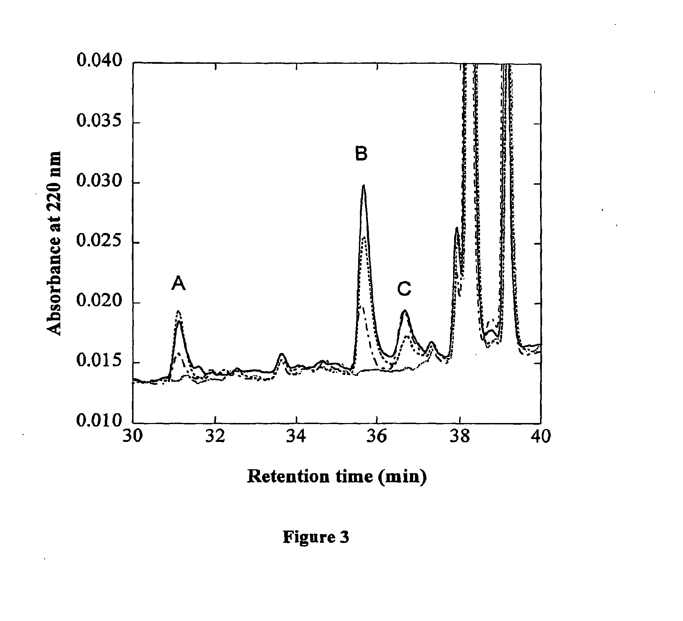

HPLC analysis of the three culture supernatants of *E*. *coli* cells expressing YvmC_{sub}, YvmCₜₕᵤ, or YvmC_{lic} resulted in three similar 220 nm-chromatograms. The three chromatograms revealed the same three resolved peaks, respectively referred hereinafter to as "peak A", peak B" and peak C", that were not found in the culture supernatant of control cells which did not express any of those genes (Figure 3). These three peaks hence corresponded to the elution of products whose synthesis was directly linked to the expression of YvmC_{sub}, YvmCₜₕᵤ or YvmC_{lic} in *E*. *coli.* Moreover, the similarity of the three 220nm-chromatograms suggested that the three homologues from *Bacillus* synthesized the same compounds. Peaks A and C corresponded to the elution of compounds which do not relate to the instant invention. Peaks B were the major peaks and they were characterized by a retention time of 35.7 min. Peaks B of the three supernatants were collected for further analysis by mass spectrometry.

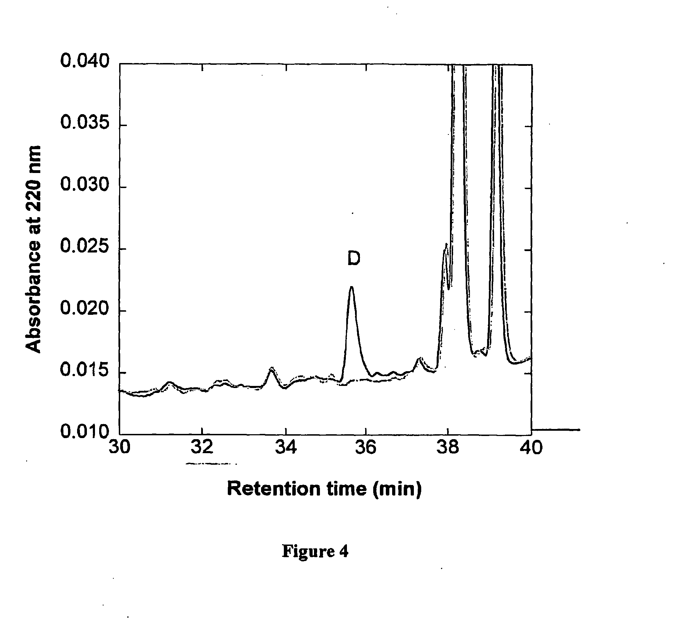

HPLC analysis of the culture supernatant of *E. coli* cells expressing Plu0297 showed only one resolved peak at 220 nm that was not found in the culture supernatant of control cells which did not express one of the genes of interest (Figure 4). This peak, hereinafter referred to as "peak D", corresponded to the elution of a product whose synthesis was directly linked to the expression of Plu0297 in *E. coli.* Peak D was characterized by a retention time of 35.7 min. Peak D was collected for further analysis by mass spectrometry.

### EXAMPLE 9: CHARACTERIZATION OF THE COMPOUNDS PRESENT IN PEAKS B AND D BY MASS SPECTROMETRY

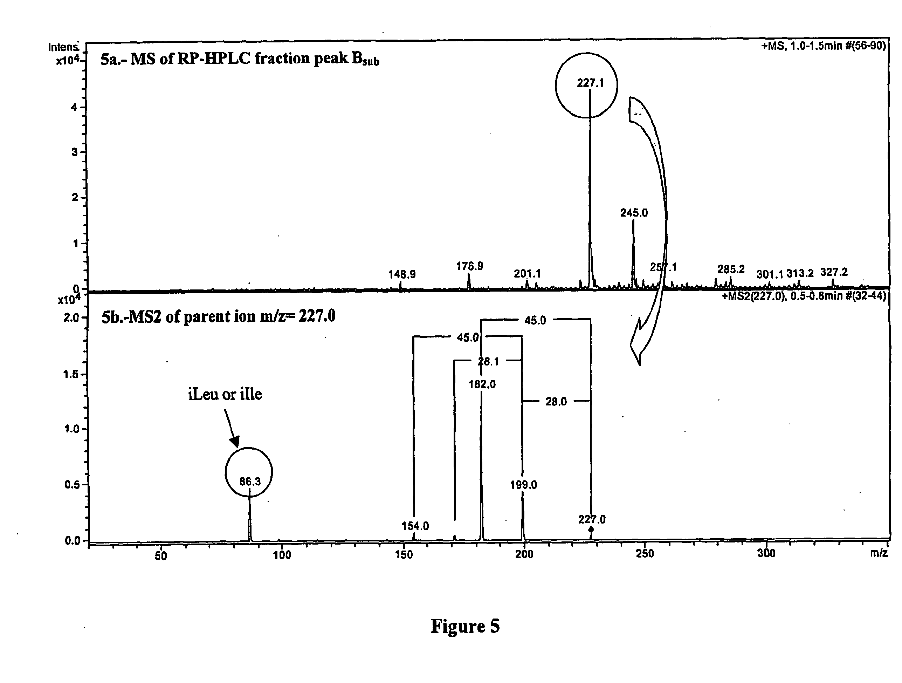

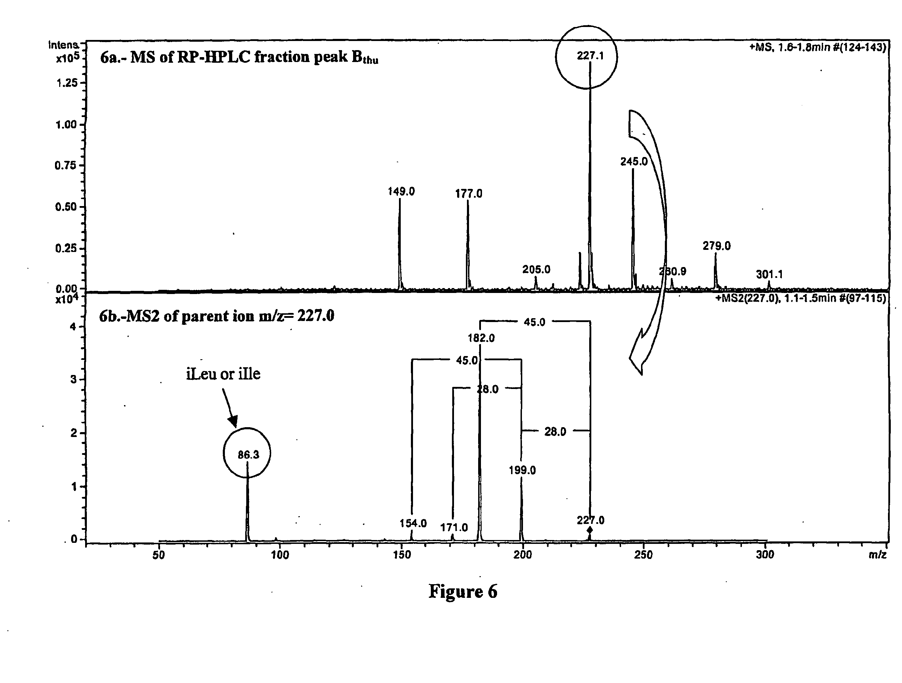

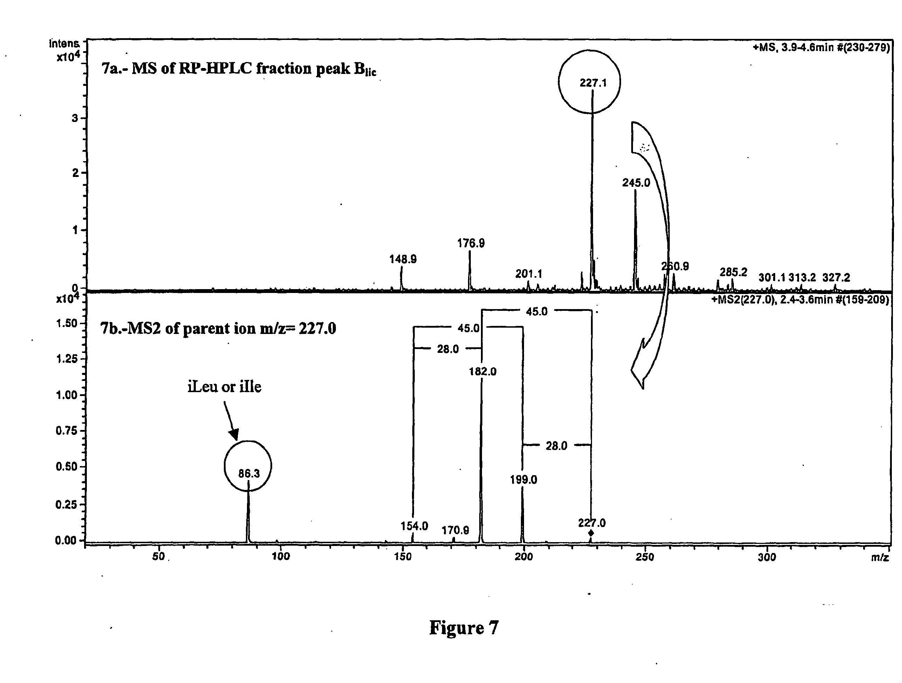

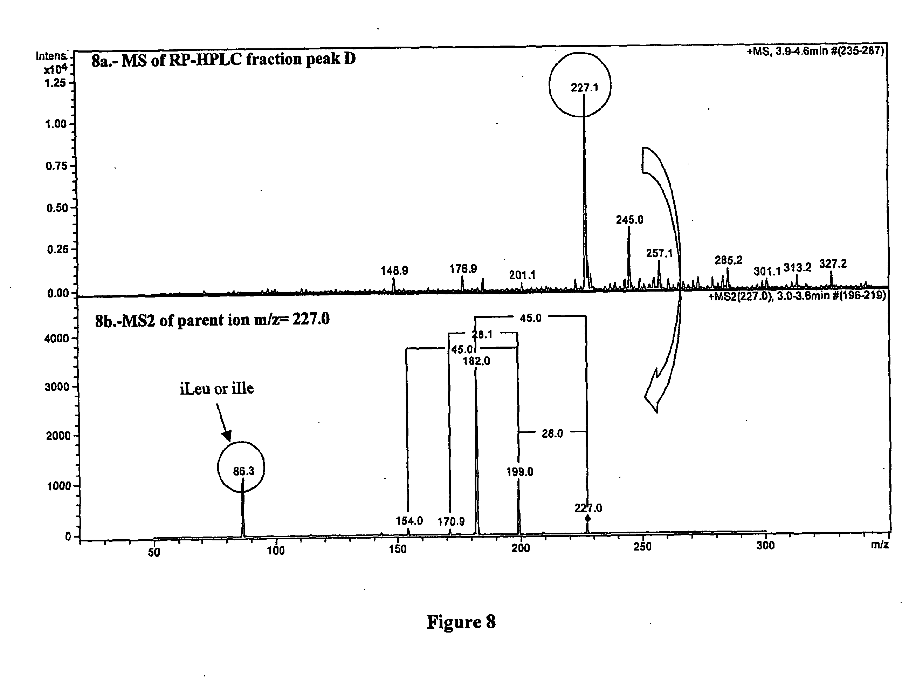

The first step in the identification of these compounds is the determination of their molecular masses. MS analyses were carried out in positive scanning mode for more sensitive detection. MS spectra of the eluted fractions corresponding to peaks B (hereinafter referred to as "peak B_{sub}" for analysis of the YvmC_{sub} supernatant, "peak Bₜₕᵤ" for analysis of the YvmCₜₕᵤ supernatant, and "peak B_{lic}" for analysis of the YvmC_{lic} supernatant) showed a major peak with the same m/z of 227.0 ± 0.1 (Figures 5a, 6a, 7a). MS spectrum of the eluted fraction corresponding to peak D also showed a major peak with an m/z of 227.0 ± 0.1 (Figure 8a). We compared this m/z value to expected mass values of natural cyclodipeptides (quoted in Table VI). It matched to four possible cyclodipeptides: cyclo(Leu-Leu), cyclo(Ile-Ile), cyclo(Leu-Ile) and cyclo(Glu-Pro).

**Table VI. Calculated monoisotopic mass (m/z) values of natural cyclodipeptides under positive mode of ESI-MS**

| **m/z of AA residue** | **Gly** | **Ala** | **Ser** | **Pro** | **Val** | **Thr** | **Cys** | **Ile** | **Leu** | **Asn** | **Asp** | **Gln** | **Lys** | **Glu** | **Met** | **His** | **Phe** | **Arg** | **Tyr** | **Trp** |
|---|---|---|---|---|---|---|---|---|---|---|---|---|---|---|---|---|---|---|---|---|
| **Gly** | 115.0 | 129.1 | 145.1 | 155.1 | 157.1 | 159.1 | 161 | 171.1 | 171.1 | 172.1 | 173 | 186.1 | 186.1 | 187.1 | 189.1 | 195.1 | 205.1 | 214.1 | 221.1 | 244.1 |
| **Ala** | | 143.1 | 159.1 | 169.1 | 171.1 | 173.1 | 175 | 185.1 | 185.1 | 186.1 | 187.1 | 200.1 | 200.1 | 201.1 | 203.1 | 209.1 | 219.1 | 228.1 | 235.1 | 258.1 |
| **Ser** | | | 175.1 | 185.1 | 187.1 | 189.1 | 191 | 201.1 | 201.1 | 202.1 | 203.1 | 216.1 | 216.1 | 217.1 | 219.1 | 225.1 | 235.1 | 244.1 | 251.1 | 274.1 |
| **Pro** | | | | 195.1 | 197.1 | 199.1 | 201.1 | 211.1 | 211.1 | 212.1 | 213.1 | 226.1 | 226.1 | 227.1 | 229.1 | 235.1 | 245.1 | 254.2 | 261.1 | 284.1 |
| **Val** | | | | | 199.1 | 201.1 | 203.1 | 213.2 | 213.2 | 214.1 | 215.1 | 228.1 | 228.2 | 229.1 | 231.1 | 237.1 | 247.1 | 256.2 | 263.1 | 286.1 |
| **Thr** | | | | | | 203.1 | 205.1 | 215.1 | 215.1 | 216.1 | 217.1 | 230.1 | 230.1 | 231.1 | 233.1 | 239.1 | 249.1 | 258.1 | 265.1 | 288.1 |
| **Cys** | | | | | | | 207 | 217.1 | 217.1 | 218.1 | 219 | 232.1 | 232.1 | 233.1 | 235 | 241.1 | 251.1 | 260.1 | 267.1 | 290.1 |
| **Ile** | | | | | | | | 227.2 | 227.2 | 228.1 | 229.1 | 242.1 | 242.2 | 243.1 | 245.1 | 251.1 | 261.2 | 270.2 | 277.1 | 300.2 |
| **Leu** | | | | | | | | | 227.2 | 228.1 | 229.1 | 242.1 | 242.2 | 243.1 | 245.1 | 251.1 | 261.2 | 270.2 | 277.1 | 300.2 |
| **Asn** | | | | | | | | | | 229.1 | 230.1 | 243.1 | 243.1 | 244.1 | 246.1 | 252.1 | 262.1 | 271.1 | 278.1 | 301.1 |
| **Asp** | | | | | | | | | | | 231.1 | 244.1 | 244.1 | 245.1 | 247.1 | 253.1 | 263.1 | 272.1 | 279.1 | 302.1 |
| **Gln** | | | | | | | | | | | | 257.1 | 257.2 | 258.1 | 260.1 | 266.1 | 276.1 | 285.2 | 292.1 | 315.1 |
| **Lys** | | | | | | | | | | | | | 257.2 | 258.1 | 260.1 | 266.2 | 276.2 | 285.2 | 292.2 | 315.2 |
| **Glu** | | | | | | | | | | | | | | 259.1 | 261.1 | 267.1 | 277.1 | 286.1 | 293.1 | 316.1 |
| **Met** | | | | | | | | | | | | | | | 263.1 | 269.1 | 279.1 | 288.1 | 295.1 | 318.1 |
| **His** | | | | | | | | | | | | | | | | 275.1 | 285.1 | 294.2 | 301.1 | 324.1 |
| **Phe** | | | | | | | | | | | | | | | | | 295.1 | 304.2 | 311.1 | 334.1 |
| **Arg** | | | | | | | | | | | | | | | | | | 313.2 | 320.2 | 343.2 |
| **Tyr** | | | | | | | | | | | | | | | | | | | 327.1 | 350.1 |
| **Trp** | | | | | | | | | | | | | | | | | | | | 373.2 |

In a second step, MSMS experiments were performed in order to elucidate the chemical structure of these compounds. As already experimented on different commercial or home-made synthetic cyclodipeptides (data not shown) and also observed on cyclodipeptides daughter ions spectra published elsewhere (Chen et al., Eur Food Research Technology, 2004, 218, 589-597 ; Stark et al., J Agric Food Chem, 2005, 53, 7222-7231), cyclodipeptide derivatives are fragmented following a characteristic pattern: (i) a sequence of neutral losses which results from cleavages of the diketopiperazine ring on either sides of the carbonyl group (loss of 28 uma corresponding to the departure of C=O group) or of the amido group (loss of 45 corresponding to CONH₃) and (ii) the presence of m/z peaks of the so-called immonium ions and of their related ions (Roepstorff et al., Biomed Mass Spectrom, 1984, 11, 601 ; Johnson et al., Anal. Chem, 1987, 59, 2621-2625) which enable to identify amino acyl residues (Table VII).

**Table VII: Immonium and related ion masses m/z used for the identification of the cyclodipeptides**

| **Residue** | **Immonium ion*** | **Related ions*** |
|---|---|---|
| **Gly** | 30 | |
| **Ala** | 44 | |
| **Ser** | 60 | |
| **Pro** | 70 | |
| **Val** | 72 | 41,55,69 |
| **Thr** | 74 | |
| **Cys** | 76 | |
| **Ile** | **86** | 44, 72 |
| **Leu** | **86** | 44, 72 |
| **Asn** | 87 | ***70*** |
| **Asp** | 88 | ***70*** |
| **Gln** | 101 | 56, 84, *129* |
| **Lys** | *101* | *70*, 84, 112, 129 |
| **Glu** | *102* | |
| **Met** | *104* | 61 |
| **His** | **110** | *82, 121, 123, 138, 166* |
| **Phe** | **120** | *91* |
| **Arg** | *129* | 59, 70, *73*, 87, 100, 112 |
| **Tyr** | **136** | *91, 107* |
| **Trp** | **159** | 77, 117, **130**, **132, 70, 171** |

| | | |
|---|---|---|
| * Bold face indicates strong signals, italic indicates weak. (Reference for this table: Falick et al., J. Am. Soc. Mass Spectrom., 1993, 4, 882-893 ; Papayannopoulos, Mass Spectrom. Rev., 1995, 14, 49-73) | | |

"Peak B_{sub}", "peak Bₜₕᵤ", "peak B_{lic}", and "peak D" fractions were then subjected to fragmentations in the ion trap mass spectrometer by isolating m/z peak at 227.0± 0.1 as parent ion. As shown in every daughter ions spectrum, MSMS fragmentation of m/z peak at 227.0± 0.1 from compounds B_{sub}, Bₜₕᵤ, B_{lic} and D (Figures 5b, 6b, 7b and 8b respectively) produces both the characteristic signature of fragmentation of diketopiperazine ring with the neutral losses of 28 and 45 and a m/z peak at 86 that could be attributed to the leucine residue or its isomass compound isoleucine. These results enabled to attribute the major m/z peak in HPLC-fractions B_{sub}, Bₜₕᵤ, B_{lic} and D to either cyclo(Leu-Leu) or cyclo(Leu-Ile) or cyclo(Ile-Ile).

### EXAMPLE 10: RECOMBINANT E. COLI EXPRESSING YvmC_{sub}, YvmCₜₕᵤ, YvmC_{lic} AND Plu0297 PRODUCE CYCLO(Leu-Leu)

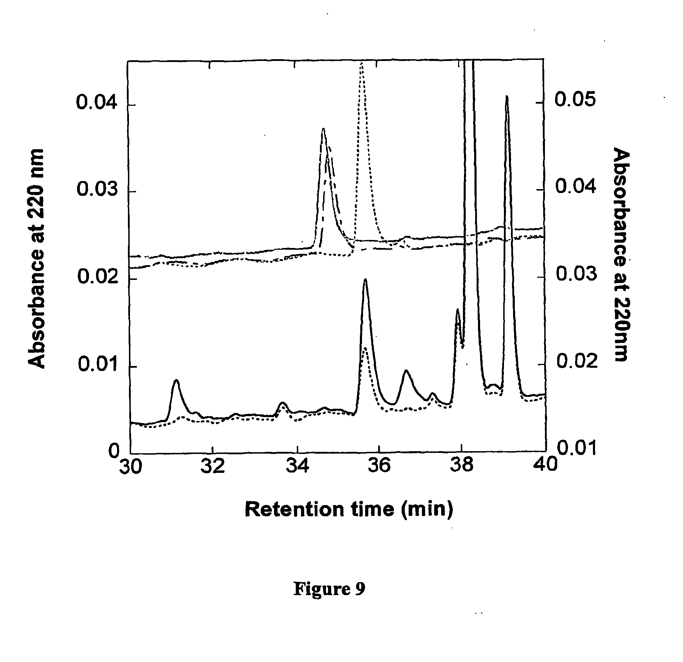

As observed previously, mass spectrometry analysis cannot differentiate cyclodipeptides containing isoleucine from those containing leucine residues. To check the chromatographic behaviours of the three different cyclodipeptides cyclo(Leu-Leu), cyclo(Leu-Ile) and cyclo(Ile-Ile) were checked in order to detect differences that could lead us to the final identification of the product synthesized by YvmC_{sub}, YvmCₜₕᵤ, YvmC_{lic} and Plu0297. HPLC-analysis at 220 nm of the three reference compounds showed that they display different retention times as cyclo(Ile-Ile) is first eluted at 34.7 min, cyclo(Ile-Leu) at 34.9 min and finally cyclo(Leu-Leu) at 35.7 min (Figure 9). The cyclodipeptides produced by YvmC_{sub} and Plu0297 were then analysed by HPLC. The 220 nm-chromatograms showed that the cyclodipeptides produced by YvmC_{sub} and Plu0297 and characterized by m/z values of 227.0± 0.1 (Figures 5a and 8a) were eluted with a retention time around 35.7 min that is similar to that of cyclo(Leu-Leu) (Figure 9). This result was confirmed by performing co-injections of reference cyclo(Leu-Leu) and culture supernatants of cells expressing YvmC_{sub} and Plu0297. Consequently, the compounds eluted in peak B_{sub} (Figures 3 & 4) and peak D (Figure 5) correspond to cyclo(Leu-Leu). Moreover, peaks Bₜₕᵤ, B_{lic} being characterized by the same retention time than that of peak B_{sub}, the corresponding eluted compounds are also cyclo(Leu-Leu).

Thus expression of YvmC_{sub}, YvmCₜₕᵤ, YvmC_{lic} or Plu0297 enzymes in *E. coli* leads to the synthesis of cyclo(Leu-Leu) cyclodipeptides found in the culture medium, demonstrating that Plu0297 and the three YvmC-like proteins are cyclo(Leu-Leu)-synthetases that can be produced in active forms in *E. coli.*

### EXAMPLE 11: IN VITRO PRODUCTION OF CYCLO(LEU-LEU) BY THE PURIFIED YvmC_{sub} PROTEIN

### Production of the purified YvmC_{sub} protein

Bacterial culture for production of the YvmC_{sub} protein was performed as already described in Example 4, except that minimal medium was replaced by LB medium (Sambrook et al., aforementioned). After induction with 0.02% arabinose, the culture was continued at 20°C for 12h. The bacterial cells were harvested by centrifugation at 4,000 g for 20 min and frozen at -80°C. Then, bacterial cells were thawed and resuspended in 1.5 ml of an extraction buffer composed of 100 mM Tris-HCl pH 8, 150 mM NaCl and 5% glycerol. Cells were broken using an Eaton press and centrifuged at 20,000 g and 4°C for 20 min. The resulting supernatant containing the soluble proteins was loaded onto a Ni²⁺-column (HisTrap HP from Amersham) equilibrated with a buffer composed of 100 mM Tris-HCl pH 8, 150 mM NaCl. The column was washed with the same buffer and submitted to a linear gradient of imidazole (from 0 to 1 M imidazole at pH 8). The YvmC_{sub} protein was eluted at around 250 mM imidazole. The purified YvmC_{sub} protein was then washed (to eliminate imidazole) and concentrated using a Vivaspin concentrator (Vivascience).

### Preparation of the soluble cell extract used for supplementation

Bacteria transformed with the empty vector pQE60 (Qiagen) were cultivated and broken as previously described. The broken cells were centrifuged at 20,000 g and 4°C for 20 min. The resulting supernatant corresponds to a soluble extract of *E. coli* cells, which does not contain cyclodipeptide synthetase.

### In vitro production of cyclo(Leu-Leu)

A 215 µl-reaction mixture comprising 6.0 mM Phe, 7.6 mM Leu, 10 mM ATP, 20 mM MgCl₂ and 25 µM of the purified YvmC_{sub} protein was supplemented with 115 µl of the previously described soluble cell extract. This mixture was incubated at 30°C for 12h. The reaction was stopped by adding TFA and submitted to a centrifugation at 20,000 g for 20 min. The supernatant was then analyzed by HPLC and HPLC-eluted fractions were characterized by mass spectrometry as described in Examples 6 and 7. As a control, the same experiment was performed under similar conditions except that the purified YvmC_{sub} was omitted.

The results clearly showed that the incubated mixture comprising the YvmC_{sub} protein contains cyclo(Leu-Leu) (an HPLC-eluted fraction at a retention time of 35.7 min with mass characteristics similar to that shown in Figure 5) whereas the incubated mixture devoid of the YvmC_{sub} protein contains no cyclodipeptide. This demonstrates that the formation of cyclo(Leu-Leu) can be performed *in vitro* with a purified cyclo(Leu-Leu) synthetase.

The procedure described for the YvmC_{sub} protein can be applied to YvmCₜₕᵤ, YvmC_{lic} and Plu0297.

## Claims

1. Use of an enzyme selected from the group consisting of the polypeptides of sequences SEQ ID N°2, SEQ ID N°4, SEQ ID N°6 and SEQ ID N°8, for the synthesis of a cyclo(Leu-Leu) cyclodipeptide.

2. Use of a polynucleotide selected from:
a) a polynucleotide encoding a cyclodipeptide synthetase as defined in claim 1;
b) a complementary polynucleotide of the polynucleotide a);
c) a polynucleotide which hybridises to polynucleotide a) or b) under stringent conditions; and
d) a polynucleotide encoding a cyclodipeptide synthethase selected from the group consisting of the polynucleotides of sequences SEQ ID N°1, SEQ ID N°3, SEQ ID N°5 and SEQ ID N°7,
for the synthesis of a cyclo(Leu-Leu) cyclodipeptide.

3. Use of a recombinant vector comprising a polynucleotide as defined in claim 2, for the synthesis of a cyclo(Leu-Leu) cyclodipeptide.

4. Use of a modified host cell comprising a polynucleotide as defined in claim 2, for the synthesis of a cyclo(Leu-Leu) cyclodipeptide.

5. Method for the synthesis of a cyclo(Leu-Leu) cyclodipeptide **characterized in that** it comprises the steps of:
(1) incubating leucine, under suitable conditions, with at least one cyclodipeptide synthetase as defined in claim 1, and
(2) recovering the cyclo(Leu-Leu) cyclodipeptide thus obtained.

6. Method for the synthesis of α,β-dehydrogenated cyclo(Leu-Leu) cyclodipeptide, **characterized in that** it comprises:
(1') incubating leucine, under suitable conditions, with a cyclodipeptide synthetase enzyme as defined in claim 1 and a purified CDO, and
(2') recovering the α,β-dehydrogenated cyclodipeptides.

7. The method according to claim 5 or claim 6, wherein step (1) or step (1') is performed in presence of Leu at a concentration between 0.1 mM and 100 mM, preferably of 1 mM to 10 mM, said cyclodipeptide synthetase at a concentration between 0.1 nM and 100 µM, preferably of 1 µM and 100 µM, in a buffer at pH of between 6 and 8, and containing a soluble extract of prokaryote cells such as *E. coli* or *Streptomyces* cells which does not produce cyclodipeptide synthetase.

8. Method according to anyone of claims 5 to 7, **characterized in that** it further comprises, prior or simultaneously with step (1) or step (1'), a step consisting in the use of a polynucleotide as defined in claim 2, for synthesizing said cyclodipeptide synthetase.

9. Method for producing cyclo(Leu-Leu) cyclodipeptide **characterized in that** it comprises the steps of:
(1') culturing a host cell as defined in claim 4 in suitable culture conditions for said host cell, and
(2') recovering the cyclo(Leu-Leu) cyclodipeptide from the culture medium.

10. Method for the synthesis of α,β-dehydrogenated cyclo(Leu-Leu) cyclodipeptide, **characterized in that** it comprises the following steps:
(1') culturing a modified host cell as defined in claim 4 in appropriate culture conditions for said host cell,
(1") incubating the cyclo(Leu-Leu) cyclodipeptide obtained from step (1') with a purified CDO, and
(2") recovering the α,β-dehydrogenated cyclo(Leu-Leu) cyclodipeptide derivative from the culture medium.

## Patentansprüche

1. Verwendung eines Enzyms, das aus der Gruppe, bestehend aus den Polypeptiden der Sequenzen SEQ ID NO:2, SEQ ID NO:4, SEQ ID NO:6 und SEQ ID NO:8, ausgewählt ist, für die Synthese eines Cyclo(Leu-Leu)cyclodipeptids.

2. Verwendung eines Nukleotids, ausgewählt aus:
a) einem Polynukleotid, das für eine Cyclodipeptidsynthetase, wie sie in Anspruch 1 definiert ist, codiert;
b) einem komplementären Polynukleotid des Polynukleotids a) ;
c) einem Polynukleotid, das unter stringenten Bedingungen an Polynukleotid a) oder b) hybridisiert, und
d) einem Polynukleotid, das für eine Cyclodipeptidsynthetase codiert, das aus der Gruppe, bestehend aus den Polynukleotiden der Sequenzen SEQ ID NO:1, SEQ ID NO:3, SEQ ID NO:5 und SEQ ID NO:7, ausgewählt ist,
für die Synthese eines Cyclo(Leu-Leu)cyclodipeptids.

3. Verwendung eines rekombinanten Vektors, der ein Polynukleotid, wie es in Anspruch 2 definiert ist, umfasst, für die Synthese eines Cyclo(Leu-Leu)cyclodipeptids.

4. Verwendung einer modifizierten Wirtszelle, die ein Polynukleotid, wie es in Anspruch 2 definiert ist, umfasst, für die Synthese eines Cyclo(Leu-Leu)cyclodipeptids.

5. Verfahren für die Synthese eines Cyclo(Leu-Leu)cyclodipeptids, **dadurch gekennzeichnet, dass** es die Schritte:
(1) Inkubieren von Leucin unter geeigneten Bedingungen mit wenigstens einer Cyclodipeptidsynthase, wie sie in Anspruch 1 definiert ist, und
(2) Gewinnen des so hergestellten Cyclo(Leu-Leu)cyclodipeptids
umfasst.

6. Verfahren für die Synthese von α,β-dehydriertem Cyclo (Leu-Leu) cyclodipeptid, **dadurch gekennzeichnet, dass** es umfasst:
(1') Inkubieren von Leucin unter geeigneten Bedingungen mit einem Cyclodipeptidsynthetase-Enzym, wie es in Anspruch 1 definiert ist, und einer gereinigten CDO und
(2') Gewinnen der α,β-dehydrierten Cyclodipeptide.

7. Verfahren gemäß Anspruch 5 oder Anspruch 6, wobei Schritt (1) oder Schritt (1') in Gegenwart von Leu mit einer Konzentration von zwischen 0,1 mM und 100 mM, vorzugsweise 1 mM bis 10 mM, der Cyclodipeptidsynthetase mit einer Konzentration von zwischen 0,1 nM und 100 µM, vorzugsweise 1 µM und 100 µM, in einem Puffer mit einem pH von zwischen 6 und 8, enthaltend einen löslichen Extrakt von prokaryoten Zellen, zum Beispiel *E. coli* oder *Streptomyces*-Zellen, die keine Cyclodipeptidsynthetase produzieren, durchgeführt wird.

8. Verfahren gemäß einem der Ansprüche 5 bis 7, **dadurch gekennzeichnet, dass** es außerdem vor oder gleichzeitig mit Schritt (1) oder Schritt (1') einen Schritt umfasst, der in der Verwendung eines Polynukleotids, wie es in Anspruch 2 definiert ist, zum Synthetisieren der Cyclodipeptidsynthetase besteht.

9. Verfahren zur Herstellung von Cyclo(Leu-Leu)cyclodipeptid, **dadurch gekennzeichnet, dass** es die Schritte:
(1') Kultivieren einer Wirtszelle, wie sie in Anspruch 4 definiert ist, unter geeigneten Kulturbedingungen für die Wirtszelle und
(2') Gewinnen des Cyclo(Leu-Leu)cyclodipeptids aus dem Kulturmedium
umfasst.

10. Verfahren für die Synthese von α,β-dehydriertem Cyclo (Leu-Leu) cyclodipeptid, **dadurch gekennzeichnet, dass** es die folgenden Schritte umfasst:
(1') Kultivieren einer modifizierten Wirtszelle, wie in Anspruch 4 definiert, unter geeigneten Kulturbedingungen für die Wirtszelle,
(1") Inkubieren des Cyclo(Leu-Leu)cyclodipeptids, das aus Schritt (1') erhalten wird, mit einer gereinigten CDO und (2 ") Gewinnen des α,β-dehydrierten Cyclo(Leu-Leu)cyclodipeptidderivats aus dem Kulturmedium.

## Revendications

1. Utilisation d'une enzyme choisie dans le groupe constitué par les polypeptides ayant les séquences SEQ ID n° 2, SEQ ID n° 4, SEQ ID n° 6 et SEQ ID n° 8, pour la synthèse d'un cyclodipeptide cyclo(Leu-Leu).

2. Utilisation d'un polynucléotide choisi parmi :
a) un polynucléotide codant pour une cyclodipeptide synthétase telle que définie dans la revendication 1 ;
b) un polynucléotide complémentaire du polynucléotide a) ;
c) un polynucléotide qui s'hybride au polynucléotide a) ou b) dans des conditions de stringence ; et
d) un polynucléotide codant pour une cyclodipeptide synthétase choisi dans le groupe constitué par les polynucléotides ayant les séquences SEQ ID n° 1, SEQ ID n° 3, SEQ ID n° 5 et SEQ ID n° 7,
pour la synthèse d'un cyclodipeptide cyclo(Leu-Leu).

3. Utilisation d'un vecteur recombinant comprenant un polynucléotide tel que défini dans la revendication 2, pour la synthèse d'un cyclodipeptide cyclo(Leu-Leu).

4. Utilisation d'une cellule hôte modifiée comprenant un polynucléotide tel que défini dans la revendication 2, pour la synthèse d'un cyclodipeptide cyclo(Leu-Leu).

5. Procédé pour la synthèse d'un cyclodipeptide cyclo(Leu-Leu) **caractérisé en ce qu'**il comprend les étapes consistant à :
(1) incuber de la leucine, dans des conditions appropriées, avec au moins une cyclodipeptide synthétase telle que définie dans la revendication 1 et
(2) récupérer le cyclodipeptide cyclo(Leu-Leu) ainsi obtenu.

6. Procédé pour la synthèse d'un cyclodipeptide cyclo(Leu-Leu) α,β-déshydrogéné, **caractérisé en ce qu'**il comprend :
(1') l'incubation de leucine, dans des conditions appropriées, avec une enzyme cyclodipeptide synthétase telle que définie dans la revendication 1 et une CDO purifiée et
(2') la récupération des cyclodipeptides α,β-déshydrogénés.

7. Procédé selon la revendication 5 ou la revendication 6, dans lequel l'étape (1) ou l'étape
(1') est effectuée en présence de Leu à une concentration comprise entre 0,1 mM et 100 mM, de préférence de 1 mM à 10 mM, de ladite cyclodipeptide synthétase à une concentration comprise entre 0,1 nM et 100 µM, de préférence de 1 µM à 100 µM, dans un tampon à un pH compris entre 6 et 8, et contenant un extrait soluble de cellules procaryotes telles que des cellules de *E. coli* ou de *Streptomyces* qui ne produisent pas de cyclodipeptide synthétase.

8. Procédé selon l'une quelconque des revendications 5 à 7, **caractérisé en ce qu'**il comprend en outre, avant ou en même temps que l'étape (1) ou l'étape (1'), une étape consistant en l'utilisation d'un polynucléotide tel que défini dans la revendication 2, pour la synthèse de ladite cyclodipeptide synthétase.

9. Procédé pour la production de cyclodipeptide cyclo(Leu-Leu) **caractérisé en ce qu'**il comprend les étapes consistant à :
(1') effectuer la culture d'une cellule hôte telle que définie dans la revendication 4 dans des conditions de culture appropriées pour ladite cellule hôte et
(2') récupérer le cyclodipeptide cyclo(Leu-Leu) à partir du milieu de culture.

10. Procédé pour la synthèse de cyclodipeptide cyclo(Leu-Leu) α,β-déshydrogéné, **caractérisé en ce qu'**il comprend les étapes suivantes :
(1') la culture d'une cellule hôte modifiée telle que définie dans la revendication 4 dans des conditions de culture appropriées pour ladite cellule hôte,
(1") l'incubation du cyclodipeptide cyclo(Leu-Leu) obtenu à partir de l'étape (1') avec une CDO purifiée et
(2") la récupération du dérivé de cyclodipeptide cyclo(Leu-Leu) α,β-déshydrogéné à partir du milieu de culture.
